# EUROPEAN PATENT SPECIFICATION

(11) **EP 2 640 182 B1**
(45) Date of publication and mention of the grant of the patent: **25.07.2018**
(21) Application number: 11841328.5
(22) Date of filing: 15.11.2011
(51) Int. Cl.: A01H 4/00, C12N 15/05, C12N 15/29, C12N 15/82, A01H 5/10, C07K 14/415, A01H 1/04

(54) **DROUGHT TOLERANT PLANTS**
TROCKENRESISTENTE PFLANZEN
PLANTES RÉSISTANTES À LA SÉCHERESSE

(30) Priority: 15.11.2010 US 413902 P
(43) Date of publication of application: 25.09.2013
(73) Proprietor: The State of Queensland acting through The Department of Agriculture and Fisheries, Brisbane QLD 4000 (AU); The Texas A&M University System, College Station, TX 77843-3369 (US)
(72) Inventor: BORRELL, Andrew Kennneth, Warwick Queensland 4370 (AU); JORDAN, David Robert, Warwick Queensland 4370 (AU); MULLET, John, College Station Texas 77845 (US); KLEIN, Patricia, Collage Station Texas 77845 (US)
(74) Representative: Dehns
(86) International application number: PCT/AU2011/001478
(87) International publication number: WO 2012/065219

(56) References cited:
- WO-A1-2004/050873
- WO-A2-03/008540
- WO-A2-2007/011771
- US-A1- 2009 094 717
- FELTUS F A ET AL: "Alignment of genetic maps and QTLs between inter and intra-specific sorghum populations", THEORETICAL AND APPLIED GENETICS ; INTERNATIONAL JOURNAL OF PLANT BREEDING RESEARCH, SPRINGER, BERLIN, DE, vol. 112, no. 7, 1 May 2006 (2006-05-01), pages 1295-1305, XP019322252, ISSN: 1432-2242, DOI: 10.1007/S00122-006-0232-3
- KEBEDE H ET AL: "Quantitative trait loci influencing drought tolerance in grain sorghum (Sorghum bicolor L. Moench)", THEORETICAL AND APPLIED GENETICS, vol. 103, no. 2-3, August 2001 (2001-08), pages 266-276, XP002722214, ISSN: 0040-5752
- CHENJIA SHEN ET AL: "Expression profile of PIN, AUX/LAX and PGP auxin transporter gene families in Sorghum?bicolor under phytohormone and abiotic stress", FEBS JOURNAL, vol. 277, no. 14, 2 June 2010 (2010-06-02) , pages 2954-2969, XP055069181, ISSN: 1742-464X, DOI: 10.1111/j.1742-4658.2010.07706.x
- MADHAVI LATHA A ET AL: "Development of transgenic pearl millet (Pennisetum glaucum (L.) R. Br.) plants resistant to downy mildew", PLANT CELL REPORTS, SPRINGER, BERLIN, DE, vol. 25, no. 9, 11 April 2006 (2006-04-11) , pages 927-935, XP019423765, ISSN: 1432-203X, DOI: 10.1007/S00299-006-0141-6
- J.-R. WANG ET AL: "Expression of PIN Genes in Rice (Oryza sativa L.): Tissue Specificity and Regulation by Hormones", MOLECULAR PLANT, vol. 2, no. 4, 1 July 2009 (2009-07-01), pages 823-831, XP055109517, ISSN: 1674-2052, DOI: 10.1093/mp/ssp023
- SHEN C. ET AL.: 'Expression profile of PIN, AUX/LAX and PGP auxin transporter gene families in Sorghum bicolor under phytohormone and abiotic stress' FEBS JOURNAL vol. 277, July 2010, pages 2954 - 2969, XP055069181
- HARRIS K. ET AL.: 'Sorghum stay-green QTL individually reduce post-flowering drought-induced leaf senescence' JOURNAL OF EXPERIMENTAL BOTANY vol. 58, no. 2, 2007, pages 327 - 338, XP055069160
- KRECEK P. ET AL.: 'The PIN-FORMED (PIN) protein family of auxin transporters' GENOME BIOLOGY vol. 10, 2009, pages 249.1 - 249.11, XP055087588
- FELTUS F.A. ET AL.: 'Alignment of genetic maps and QTLs between inter- and intra- specific sorghum populations' THEORETICAL AND APPLIED GENETICS vol. 112, no. 7, 2006, pages 1295 - 1305, XP019322252
- K. HARRIS ET AL: "Sorghum stay-green QTL individually reduce post-flowering drought-induced leaf senescence", JOURNAL OF EXPERIMENTAL BOTANY, vol. 58, no. 2, 6 November 2006 (2006-11-06), pages 327-338, XP055069160, ISSN: 0022-0957, DOI: 10.1093/jxb/erl225

## Description

### FIELD

The present specification teaches the generation of drought-tolerant plants. The present disclosure enables manipulation of a phenotypic characteristic referred to herein as "stay-green" to generate drought-tolerant plants by recombinant, mutagenic and/or breeding and selection methods.

### BACKGROUND

Bibliographic details of the publications referred to by author in this specification are collected alphabetically at the end of the description.

An increasing human population necessitates improvements in crop productivity. This has been a major goal for plant breeders and geneticists. One approach to improving crop productivity involves the selection of plant traits which facilitate higher grain yield and stability (Springer, Nature Genetics 42:475-476, 2010). This approach has been referred to as the "Green Revolution". Other approaches include the development of ideal plant architectures which have, for example, led to the identification of a quantitative trait locus (QTL) which encodes squamosa promoter binding protein-like 14 (OsSPL14) in rice and which facilitates improved rice yield (Jiao et al., Nature Genetics 42:541-544, 2010; Miura et al., Nature Genetics 42:545-549, 2010).

Drought is the single most important constraint to cereal production worldwide. Sorghum is a repository of drought resistance mechanisms, which include C₄ photosynthesis, deep roots and thick leaf wax which enable growth in hot and dry environments. Drought tolerance makes sorghum especially important in dry regions such as sub-Saharan Africa, western-central India, north-eastern Australia, and the southern plains of the US. With increasing pressure on the availability of scarce water resources, the identification of traits associated with grain yield under drought conditions becomes more important.

The drought adaptation mechanism identified in sorghum which results in the retention of green leaves for longer periods during grain filling under drought is known as 'stay-green'. Stay-green has been associated with high grain yield under post-anthesis drought in sorghum (Borrell et al., Crop Sci. 40:1037-1048, 2000b; Kassahun et al., Euphytica 72:351-362, 2010), wheat (*Triticum aestivum* L.) [Spano et al., J. Exp. Bot. 54:1415-1420, 2003; Christopher et al., Aust. J. Agric. Res. 59:354-364, 2008], rice (*Oryza sativa* L.) [Kashiwagi et al., Plant Physiology and Biochemistry 44:152-157, 2006] and maize (*Zea mays* L.) [Zheng et al., Plant Breed 128:54-62, 2009]. In addition, it may indirectly affect grain yield under drought by improving charcoal rot (*Macrophomina phaseolina* [Tassi] Goid.) resistance (Tenkouano et al., Theor. Appl. Genet. 85:644-648, 1993; Garud et al., Int. Sorghum and Millets Newsl. 43:63-65, 2002). This reduces lodging (Reddy et al., Euphytica 159:191-198, 2008), allowing plant breeders to exploit the positive association between plant height and grain yield (Jordan et al., Theor. Appl. Genet. 106:559-567, 2003). Stay-green has been an important selection criterion for sorghum breeding programs targeting drought adaptation in both the US (Rosenow et al., Agric. Water Manag. 7:207-222, 1983) and Australia (Henzell et al., Australia Int. Sorghum and Millets Newsl. 38:1-9, 1997).

A considerable body of physiological evidence is mounting in support of this trait (Borrell et al., Crop Sci. 40:1026-1037, 2000a; Borrell and Hammer, Crop Sci. 40:1295-1307, 2000; Harris et al., J. Exp. Bot. 58:327-338, 2007; Christopher *et al.,* 2008 *supra;* Van Oosterom et al., Field Crops Res. 115:19-28, 2010a and Van Oosterom et al., Field Crops Res. 115:29-38, 2010b). Although this drought resistance mechanism has been utilized by sorghum breeders in the US and Australia for over 25 years, and the broad physiological basis of the trait is becoming better understood, the causal mechanisms and the genetic loci involved have hitherto been unknown.

Under water-limiting conditions, grain yield is a function of transpiration (T), transpiration efficiency (TE), and harvest index (HI) [Passioura, J. Aust. Inst. Agric. Sci. 43:117-120, 1977]. Within this framework, grain yield is linked to post-anthesis T (Turner, J. Exp. Bot. 55:2413-2425, 2004; Manschadi et al., Funct. Plant. Biol. 33:823-837, 2006), because HI increases with the fraction of total crop T used after anthesis (Passioura, 1977 *supra*; Sadras and Connor, Field Crops Res. 26:227-239, 1991; Hammer, Agric. Sci. 19:16-22, 2006). Increased post-anthesis T is associated with reduced drought stress around anthesis, which can positively affect crop growth rate at anthesis of cereals and hence grain number (Andrade et al., Crop Sci. 42:1173-1179, 2002; Van Oosterom and Hammer, Field Crops Res. 108:259-268, 2008). If the total amount of available water is limited, post-anthesis T can be increased by restricting pre-anthesis T. This can be achieved by restricting canopy size, either genetically or through crop management. However, a smaller canopy will only reduce total T if its TE is not compromised. Significant genotypic differences in TE have been reported for sorghum (Hammer et al., Aust. J. Agric. Res. 48:649-655, 1997; Henderson et al., Aust. J. Plant Physiol. 25:111-123, 1998; Mortlock and Hammer, J. Crop Prod. 2:265-286, 1999; Xin et al., Field Crops Res. 111:74-80, 2009). Alternatively, post-anthesis water use can be increased by increasing the total amount of water accessed by the crop, either through deeper rooting or reduced lower limit of water extraction (Manschadi *et al.,* 2006 *supra*).

The stay-green trait affects a number of the above processes in sorghum. First, stay-green reduces water use during the pre-anthesis period by restricting canopy size (*via* reduced tillering and smaller leaves).

Second, stay-green improves water accessibility by increasing the root:shoot ratio. There is some experimental evidence for better water extraction in stay-green lines, although more research is required. These root responses could also be explained by enhanced auxin transport (Wang et al., Molecular Plant 2(4):823-831, 2009). Third, stay-green increases the greenness of leaves at anthesis, effectively increasing photosynthetic capacity, and, therefore, TE (providing that photosynthesis increases proportionately more than conductance). The increase in leaf greenness is an indirect affect of reduced leaf mass, i.e. nitrogen is concentrated in the leaf.

Producing more food with less water is one of the major challenges currently facing humanity. There is a real and urgent need in both developing and developed countries to identify the genes and gene networks controlling drought adaptation in crop plants. This enables increased drought adaptation in a wide range of crop species grown in water-limited environments worldwide.

### SUMMARY

The present disclosure teaches quantitative trait loci (QTLs) associated with and/or which otherwise facilitate the stay-green phenotype. The QTLs are referred to herein as "stay-green (Stg) X" wherein X is a numeral increasing from 1 which represents the region on a chromosome associated with the stay-green phenotype.

As taught herein, the QTLs identify genetic regions on sorghum which carry loci which encode one to a number of proteins or regulatory agents such as microRNAs which facilitate the stay-green phenotype. Modulation of expression of one or more these loci in a crop plant generates a canopy architecture which facilitates a shift in water use by the plant to the post-anthesis period or increased accessibility of water during crop growth or increased transpiration efficiency thereby increasing harvest index (HI) and grain yield under water-limited conditions.

The loci encode PIN proteins which are associated with auxins. PIN proteins are auxin efflux carriers which contain transmembrane domains and are mainly localized in the plasma membranes. The term "PIN" is derived from "pin-like inflorescence".

The term "SbPINn" is used to describe such a gene in sorghum wherein n is a numeral defining the auxin efflux carrier component and n is 1 through 11. Reference to "SbPINn" includes its homologs and orthologs in other plants. Examples of SbPINn loci in sorghum include those listed in Table 1A such as but not limited to SbPIN4 and SbPIN2. Modulation of expression of a PIN or expression of a PIN with a particular polymorphic variation is taught herein to facilitate exhibition of the stay-green phenotype. The present disclosure also teaches PINs from other plants such as rice. The letter prefix of the PIN specifies its source (e.g. Sb, sorghum; Os, rice; etc). The location of a SbPIN in sorghum is determined by gene ID number (See Table 1A). As examples, SbPIN4 corresponds to OsPIN5 and SbPIN2 corresponds to OsPIN3a.

SbPIN4 and SbPIN2 are examples of SbPINs taught herein to be responsible for the stay-green trait in sorghum associated with Stg1, and Stg2, respectively, resulting in a range of phenotypes that confer drought adaptation via decreased water use until anthesis (due to reduced tillering and smaller leaves), increased water accessibility (due to enhanced root:shoot ratio), increased transpiration efficiency under mild water deficit (due to higher leaf nitrogen concentration), increased biomass per leaf area under terminal water deficit (due to increased transpiration per leaf area) and increased grain yield, grain size and lodging resistance. Other examples are listed in Table 1A and include their equivalents in other plants.

The present disclosure provides a method for generating a genetically modified plant which uses water more efficiently than a non-genetically modified plant of the same species, the method comprising introducing by genetic engineering using recombinant means genetic material which encodes a sorghum (Sb) pin-like inflorescence (PIN) 4 protein to confer a stay-green phenotype, which phenotype includes a shift in water use to the post-anthesis period or increased accessibility of water during crop growth or increased transpiration efficiency resulting in increased harvest index and grain yield under water-limited conditions, wherein said SbPIN4 protein is encoded by Sorghum Gene ID Sb03g037350 from bp 65310051 to bp 65313194.

Also provided herein is the use of a genetic material which encodes a SbPIN4 protein for generating by recombinant means a genetically modified plant which uses water more efficiently than a non-genetically modified plant of the same species, wherein the said genetically modified plant has a stay-green phenotype which includes a shift in water use to the post-anthesis period or increased accessibility of water during crop growth or increased transpiration efficiency resulting in increased harvest index and grain yield under water-limited conditions, and wherein said SbPIN4 protein is encoded by Sorghum Gene ID Sb03g037350 from bp 65310051 to bp 65313194.

The following abbreviations are used in the subject specification:
- CI,: confidence interval
- CWU,: crop water use
- DW,: dry weight
- GLA,: green leaf area
- HD,: high density
- HI,: harvest index
- HT,: high tillering
- HW,: high water
- HWHD,: high water, high density (intermediate water stress)
- HWLD,: high water, low density (least water stressed)
- LA,: leaf area
- LD,: low density
- LT,: low tillering
- LW,: low water
- LWHD,: low water, high density (most water stressed)
- LWLD,: low water, low density (intermediate water stress)
- NIL,: Near-isogenic line
- OsPIN,: PIN from rice
- PAB,: post-anthesis biomass
- PASM,: post-anthesis stem mass
- PIN,: pin-like inflorescence
- PPBR,: pre:post anthesis biomass ratio
- QTL,: quantitative trait locus
- ROS,: rain-out shelter
- RWC,: relative water content
- SbPIN,: PIN from sorghum
- SLW,: specific leaf weight
- SML,: statistical machine learning
- Stg,: stay-green
- T,: transpiration
- T2,: tiller in the axil of leaf 2
- T3,: tiller in the axil of leaf 3
- T4,: tiller in the axil of leaf 4
- T5,: tiller in the axil of leaf 5
- T6,: tiller in the axil of leaf 6
- TE,: transpiration efficiency
- TS,: terminal stress
- VPD,: vapor pressure deficit
- WW,: well watered

Table 1A provides information on PINs from sorghum and rice.

**Table 1A**

| Sorghum Stg QTL details | | | | | | | | | | | | | | |
|---|---|---|---|---|---|---|---|---|---|---|---|---|---|---|
| Sorghum Gene ID | Rice homologue | sorghum | Chromosome | bp(start) | bp (end) | Length(bp) | predicted cM | LOD | R2 | Pop | Publication | Source of allele | Published symbol | Stg QTL summary |
| Sb02g0292 10 | OsPIN6 | SbPIN1 | SBI-02 | 64347 327 | 64350 341 | 3014 | 144.3023 044 | 1.9 | 10 .7 | B35/Tx70 00 | Subudhi et al 2000 | B35 | stg3 | Stg3B |
| | | | | | | | | 2.5 | 4. 9 | N13/E36-1 | Hausmann et al 2002 | N13 | %GL15 #3 | |
| | | | | | | | | 3 | 5. 8 | N13/E36-1 | Hausmann et al 2002 | N13 | %GL30 #5 | |
| | | | | | | | | 4.9 | 9. 5 | N13/E36-1 | Hausmann et al 2002 | N13 | %GL45 #4 | |
| Sb03g0293 20 | OsPIN3a | SbPIN2 | SBI-03 | 57449 784 | 57453 744 | 3960 | 88.78882 569 | 2.63 | 10 .2 | SC56/Tx7 000 | Kebede et al 2001 | SC56 | Stg A | Stg2 |
| | | | | | | | | 2.65 | 14 | B35/Tx70 00 | Subudhi et al 2000 | B35 | stg2 | |
| | | | | | | | | 2.65 | 6. 1 | 296B/IS1 8551 | Srinivas et al 2009 | 296B | QGlaa-sbi03 | |
| | | | | | | | | 2.9 | 7. 5 | 296B/IS1 8551 | Srinivas et al 2009 | 296B | QPglam-sbi03 | |
| | | | | | | | | 3.66 | 19 .9 | B35/Tx70 00 | Subudhi et al 2000 | B35 | stg2 | |
| | | | | | | | | 5.52 | 29 .2 | B35/Tx70 00 | Subudhi et al 2000 | B35 | stg2 | |
| | | | | | | | | 5.44 | 22 .6 | B35/Tx70 00 | Subudhi et al 2000 | Tx7000 | stg2 | |
| | | | | | | | | 5.6 | 24 .8 | B35/Tx70 00 | Xu et al 2000 | Tx7000 | Chl2 | |
| | | | | | | | | 6.23 | 30 .3 | B35/Tx70 00 | Xu et al 2000 | B35 | Stg2 | |
| | | | | | | | | 6.6 | 28 .6 | B35/Tx43 0 | Crasta et al 1999 | B35 | SGA | |
| | | | | | | | | 2.8 | 5. 6 | N13/E36-1 | Hausmann et al 2002 | E36-1 | %GL45 #5 | |
| Sb03g0328 50 | OsPIN1 | SbPIN3 | SBI-03 | 61297 480 | 61299 969 | 2489 | 115.1215 502 | 2.69 | 12 | B35/Tx70 00 | Xu et al 2000 | Tx7000 | Chl1 | Sto1 (broader QTL) |
| | | | | | | | | 4.59 | 19 .6 | B35/Tx70 00 | Xu et al 2000 | B35 | Stg1 | |
| | | | | | | | | 3.18 | 15 .4 | B35/Tx70 00 | Subudhi et al 2000 | B35 | Stg1 | |
| | | | | | | | | 3.61 | 18 .1 | B35/Tx70 00 | Subudhi et al 2000 | B35 | Stg1 | |
| | | | | | | | | 14.9 | 26 .3 | IS9830/E 36-1 | Hausmann et al 2002 | IS9830 | %GL15 #1 | |
| | | | | | | | | 6.5 | 12 .4 | IS9830/E 36-1 | Hausmann et al 2002 | IS9830 | %GL30 #2 | |
| Sb03g0373 50 | OsPIN5 | SbPIN4 | SBI-03 | 65310 051 | 65313 194 | 3143 | 129.5972 557 | Stg1 (fine-mapped QTL) | | | | | | Stg1 (fine-mapped QTL) |
| Sb03g0439 60 | OsPIN6 | SbPIN5 | SBI-03 | 71204 119 | 71206 483 | 2364 | 152.8989 678 | | | | | | | - |
| Sb04g0281 70 | OsPIN1 | SbPIN6 | SBI-04 | 58261 350 | 58264 959 | 3609 | 107.0875 147 | 3.63 | 13 .4 | SC56/Tx7 000 | Kebede et al 2001 | SC56 | Stg C.1 | smIQTL and near StgC.1 |
| | | | | | | | | 3.1 | 6. 1 | IS9830/E 36-1 | Hausmann et al 2002 | IS9830 | %GL15 #2 | |
| | | | | | | | | 2.8 | 5. 5 | IS9830/E 36-1 | Hausmann et al 2002 | IS9830 | %GL30 #3 | |
| | | | | | | | | 2.6 | 5. 1 | IS9830/E 36-1 | Hausmann et al 2002 | IS9830 | %GL45 #4 | |
| | | | | | | | | 4.11 | 15 .1 | SC56/Tx7 000 | Kebede et al 2001 | Tx7000 | Stg C.2 | |
| Sb05g0021 50 | OsPIN1b | SbPIN7 | SBI-05 | 23044 07 | 23076 30 | 3223 | 17.31415 415 | sml QTL only | | | | | | smIQTL |
| Sb07g0263 70 | OsPIN4 | SbPIN8 | SBI-07 | 61560 708 | 61563 133 | 2425 | 123.6163 44 | 2.8 | 5. 6 | N13/E36-1 | Hausmann et al 2002 | N13 | %GL15 #5 | Hausmann QTL and smIQTL |
| | | | | | | | | 3.4 | 6. 7 | N13/E36-1 | Hausmann et al 2002 | N13 | %GL30 #7 | |
| | | | | | | | | 2.9 | 5. 8 | N13/E36-1 | Hausmann et al 2002 | N13 | %GL45 #8 | |
| Sb10g0044 30 | OsPIN1 | SbPIN9 | SBI-10 | 39151 01 | 39175 19 | 2418 | 32.96657 613 | 3.65 | 13 .8 | SC56/Tx7 000 | Kebede et al 2001 | Tx7000 | Stg B | Kebede QTL |
| Sb10g0082 90 | OsPIN1c | SbPIN10 | SBI-10 | 84384 81 | 84415 08 | 3027 | 45.84731 059 | 2.76 | 11 .2 | QL39/QL 41 | Tao et al 2000 | QL41 | Stgl | Stgl |
| Sb10g0263 00 | OsPIN2 | SbPIN11 | SBI-10 | 55747 009 | 55751 104 | 4095 | 82.37617 984 | 2.7 | 5. 5 | N13/E36-1 | Hausmann et al 2002 | E36-1 | %GL30#8 | Hausmann & Crasta QTL |
| | | | | | | | | 2.6 | 5. 2 | N13/E36-1 | Hausmann et al 2002 | E36-1 | %GL45 #9 | |

### BRIEF DESCRIPTION OF THE FIGURES

**Figure 1** is a graphical representation showing the relation between culms per m² and green leaf area at anthesis in a range of near-isogenic lines containing various Stg introgressions.
**Figure 2** is a graphical representation showing the relation between culms per m² and green leaf area at anthesis for a range of Stg introgressions in a RTx7000 background grown under two crop densities (LD = 10 plants/m²; HD = 20 plants/m²).
**Figure 3** is a graphical representation showing the histogram of predicted values for culms per plant in the *Stg1* fine-mapping population averaged over three seasons.
**Figure 4** is a tabulated representation showing a histogram of culms per plant at 44 DAE for five genotypes grown under two water regimes. The genotypes comprise RTx7000 (recurrent parent), 6078-1 (donor parent), and three selections from the Stgl fine-mapping population. HWLD = high water, low density (10 plants/m²). LWLD = low water, low density (10 plants/m²).
**Figure 5** is a graphical representation showing the phenotypic variation in the Stg1 fine-mapping population for presence of T2.
**Figure 6** is a graphical representation showing the phenotypic variation in the Stg1 fine-mapping population for presence of T3.
**Figure 7** is a representation showing a histogram of T2 presence for eight high-tillering and eight low-tillering recombinants from the Stgl fine-mapping population.
**Figure 8** is a representation showing a histogram of total tiller number per plant for five high-tillering and three low-tillering recombinants from the Stgl fine-mapping population. A value of 2.5 was chosen as the arbitrary cut-off between high and low tillering.
**Figures 9A through D** are graphical representations showing the leaf size distribution of mainstem and tillers for RTx7000 and 6078-1 (*Stg1* NIL) grown in lysimeters under low and high VPD conditions.
**Figure 10** is a graphical representation showing the mainstem leaf size distributions of RTx7000, 6078-1 (Stg1 NIL) and three recombinants from the Stg1 fine-mapping population grown under water-limited and high-density conditions in the field (HD = 20 plants/m²).

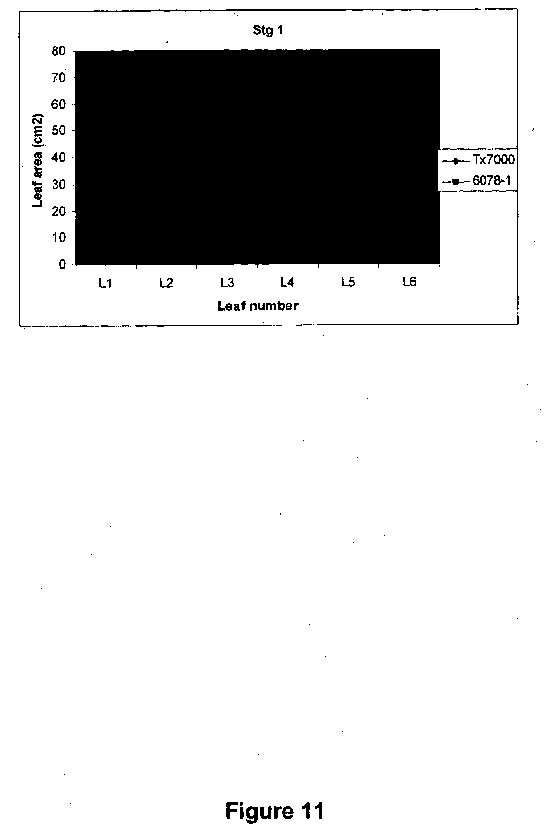

**Figure 11** is a graphical representation showing the leaf size distribution (L1-6) for the parents of the Stg1 fine-mapping population grown in an igloo.
**Figure 12** is a graphical representation showing the leaf length distribution (LI-6) for the parents of the Stg1 fine-mapping population grown in an igloo.
**Figure 13** is a graphical representation showing the leaf width distribution (LI-6) for the parents of the Stg1 fine-mapping population grown in an igloo.
**Figure 14** is a graphical representation showing the leaf size distribution (l1-11) for the parents of the Stg1 fine-mapping population grown in an igloo.
**Figure 15** is a graphical representation showing the leaf length distribution (L1-10) for the parents of the Stg1 fine-mapping population grown in an igloo.
**Figure 16** is a representation showing a histogram of phenotypic variation for L10 length in a subset of the Stg1 fine-mapping population grown in an igloo.
**Figure 17** is a diagrammatic representation showing that increased water availability at anthesis is achieved *via* reduced water use due to two mechanisms (reduced tillering and smaller leaves) in plants containing the Stgl region.
**Figure 18** is a representation showing that canopy size is modulated by both constitutive and adaptive responses controlled by a gene(s) in the Stg1 region.
**Figure 19** is a graphical representation showing the mainstem leaf size distributions of RTx7000, 6078-1 (Stg1 NIL) and three recombinants from the Stg1 fine-mapping population grown under water-limited and high-density conditions in the field (HD = 20 plants/m²).
**Figure 20** is a graphical representation showing the relation between the area of leaf 12 and the total green leaf area at anthesis for the two parents (6078-1 and RTx7000) and three recombinants from the Stg1 fine-mapping population.
**Figure 21** is a graphical representation showing the relation between total green leaf area (cm²/m²) and crop water use (mm) at anthesis for the two parents (6078-1 and RTx7000) and three recombinants from the Stg1 fine-mapping population.
**Figure 22** is a graphical representation showing the relation between green leaf area and water use (T) in four Stg QTL and the recurrent parent (RTx7000) in lysimetry studies under two levels of VPD.
**Figure 23** is a graphical representation showing a histogram of phenotypic variation for the "root:shoot ratio" at L6 in the Stgl fine-mapping population grown in an igloo.
**Figure 24** is a graphical representation showing the temporal pattern of cumulative crop water use for RTx7000 and Stg1 grown under low-water and low-density (20 plants/m²) conditions. The vertical line marks anthesis.
**Figure 25** is a graphical representation showing the relation between the length (mm) and greenness (SPAD) of leaf 10 in the Stg1 fine-mapping propulation grown in an igloo.
**Figure 26** is a graphical representation showing the relation between leaf greenness (SPAD) and leaf photosynthesis in a subset of lines from the Stg1 fine-mapping population, including the parents.
**Figure 27** is a graphical representation showing the relation between leaf greenness (SPAD) and WUE (Licor) in a subset of lines from the Stg1 fine-mapping population, including the parents.
**Figure 28** is a graphical representation showing the relation between leaf greenness (SPAD) and WUE (Licor) in four Stg Nils (Stg1, Stg2, Stg3 and Stg4) and the recurrent parent (RTx7000).
**Figure 29** is a graphical representation showing the relation between transpiration per leaf area and transpiration efficiency in four Stg QTL and the recurrent parent (RTx7000) in lysimetry studies under two levels of VPD.
**Figure 30** is a graphical representation showing the relation between CWU (mm) before and after anthesis in a subset of lines from the Stg1 fine-mapping population, including the parents, grown under high density (HD) and low density (HD) conditions.
**Figures 31A and B** are graphical representations showing patterns of cumulative water use for Stg1 and RTx7000 grown under LWHD and conditions.
**Figure 32** is a graphical representation showing the relation between CWU (mm) before and after anthesis in four Stg QTL and the recurrent parent (RTx7000) grown under low water (LW) and low density (LD) conditions.
**Figure 33** is a graphical representation showing the relation between PPBR and PAB in four Stg QTL and the recurrent parent (RTx7000) grown under water-limited conditions at two crop densities (LD = 10 plants/m²; HD = 20 plants/m²).
**Figure 34** is a graphical representation showing the relation between GLAA and PPBR in four Stg QTL and the recurrent parent (RTx7000) grown under water-limited conditions at two crop densities (LD = 10 plants/m²; HD = 20 plants/m²).
**Figure 35** is a graphical representation showing the relation between GLAA and PASB in four Stg QTL and the recurrent parent (RTx7000) grown under water-limited conditions at two crop densities (LD = 10 plants/m²; HD = 20 plants/m²).
**Figure 36** is a graphical representation showing the relation between PPBR and PAB in four Stg QTL and the recurrent parent (RTx7000) grown under water-limited conditions at two crop densities (LD = 10 plants/m²; HD = 20 plants/m²).
**Figure 37** is a graphical representation showing the relation between PPBR and PASM in four Stg QTL and the recurrent parent (RTx7000) grown under water-limited conditions at two crop densities (LD = 10 plants/m²; HD = 20 plants/m²).
**Figure 38** is a graphical representation showing the relation between PPBR and grain yield in four Stg QTL and the recurrent parent (RTx7000) grown under water-limited conditions at two crop densities (LD = 10 plants/m²; HD = 20 plants/m²).
**Figure 39** is a graphical representation showing the relation between RWC at mid-grain filling (FL-2) and the relative rate of leaf senescence in four Stg QTL and the recurrent parent (RTx7000) grown under water-limited conditions at two crop densities (LD = 10 plants/m²; HD = 20 plants/m²).
**Figure 40** is a graphical representation showing the relation between relative rate of leaf senescence and green leaf area at maturity in four Stg QTL and the recurrent parent (RTx7000) grown under water-limited conditions at two crop densities (LD = 10 plants/m²; HD = 20 plants/m²).
**Figure 41** is a graphical representation showing the relation between relative water content (RWC) at mid-grain filling (FL-2) and stem mass at maturity in four Stg QTL and the recurrent parent (RTx7000) grown under water-limited conditions at two crop densities (LD = 10 plants/m²; HD = 20 plants/m²).
**Figure 42** is a graphical representation showing the relation between post-anthesis stem mass (PASM) and post-anthesis biomass (PAB) in four Stg QTL and the recurrent parent (RTx7000) grown under water-limited conditions at two crop densities (LD = 10 plants/m²; HD = 20 plants/m²).
**Figure 43** is a graphical representation showing the relation between post-anthesis stem biomass and grain yield in four Stg QTL and the recurrent parent (RTx7000) grown under water-limited conditions at two crop densities (LD = 10 plants/m² ; HD = 20 plants/m²).
**Figure 44** is a graphical representation showing the relation between between post-anthesis stem mass (PASM) and post-anthesis biomass (PAB) in four Stg1 QTL and the recurrent parent (RTx7000) grown under water-limited conditions at two crop densities grown in an experiment in 2005 (LD = 10 plants/m²; HD = 20 plants/m²).
**Figure 45** is a graphical representation showing the relation between post-anthesis stem mass (PASM) and grain yield in four Stg QTL and the recurrent parent (RTx7000) grown under water-limited conditions at two crop densities (LD = 10 plants/m²; HD = 20 plants/m²) grown in an experiment in 2005.
**Figure 46** is a graphical representation showing the relation between post-anthesis stem mass (PASM) and post-anthesis biomass (PAB) in various Stg1 fine-mapping lines and the recurrent parent (RTx7000) grown under water-limited conditions at two crop densities (LD = 10 plants/m²; HD = 20 plants/m²).
**Figure 47** is a graphical representation showing the relation between relative water content (RWC) at mid-grain filling (FL-2) and grain yield in various combinations of Stg QTL and the recurrent parent (RTx7000) grown under water-limited conditions at two crop densities (LD = 10 plants/m2; HD = 20 plants/m2) in an experiment grown in 2004.
**Figure 48** is a graphical representation showing the relation between leaf water potential (LWP) of FL-2 at mid-grain filling (bars) and grain yield (g/m2) in the Stg1 QTL (6078-1) and the recurrent parent (RTx7000) grown under water-limited conditions at two crop densities (LD = 10 plants/m²; HD = 20 plants/m²).
**Figure 49** is a graphical representation showing the relation between PPBR and CWU during grain filling in four Stg QTL and the recurrent parent (RTx7000) grown under water-limited conditions at two crop densities (LD = 10 plants/m² ; HD = 20 plants/m²).
**Figure 50** is a graphical representation showing the relation between CWU during grain filling (mm) and grain yield (g/m²) in four Stg QTL and the recurrent parent (RTx7000) grown under water-limited conditions at two crop densities (LD = 10 plants/m²; HD = 20 plants/m²).
**Figure 51** is a graphical representation showing the relation between PPBR and grain yield in four Stg QTL and the recurrent parent (RTx7000) grown under water-limited conditions at two crop densities (LD = 10 plants/m ²; HD = 20 plants/m²).
**Figure 52** is a graphical representation showing the relation between CWU during grain filling (mm) and grain size (mg) in four Stg QTL and the recurrent parent (RTx7000) grown under water-limited conditions at two crop densities (LD = 10 plants/m²; HD = 20 plants/m²).
**Figure 53** is a graphical representation showing the relation between PPBR and grain size in four Stg QTL and the recurrent partent (RTx7000) grown under water-limited conditions at two crop densitities (LD = 10 plants/m²; HD = 20 plants/m²).
**Figure 54** is a graphical representation showing the relation between PPBR and CWU during grain filling in various Stg1 fine-mapping lines and the recurrent parent (RTx7000) grown under water-limited conditions at two crop densities (LD = 10 plants/m2; HD = 20 plants/m2).
**Figure 55** is a graphical representation showing the relation between CWU during grain filling (mm) and grain yield (g/m2) in various Stg1 fine-mapping lines and the recurrent parent (RTx7000) grown under water-limited conditions at two crop densities (LD = 10 plants/m2; HD = 20 plants/m2).
**Figures 56A through C** are graphical representations showing results from running a sorghum crop simulation model using the generic variety Buster with the usual 2 tillers/plant (HT) versus a Buster with only 1 tiller/plant (LT) in a well-watered (WW) and a terminally stressed (TS) virtual environment. For both virtual environments the following parameters were chosen: planting density 5 plants/m2 with 1m row spacing; soil depth = 1800mm; soil PAWC = 324mm; N non-limiting.
**Figure 57A** is a graphical representation of differential expression of SbPIN4 (Stg1 candidate) under well-watered conditions. Under well-watered conditions, this gene is down-regulated in young root tips Tx642 and Stg1 NIL compared to Tx7000.
**Figure 57B** is a graphical representation of differential expression of SbPIN4 (Stg1 candidate) under water-deficient conditions. Under water-deficient conditions, this gene is up-regulated in most tissues, but especially in expanding leaves of Tx642 and Stg1 NIL compared to Tx7000.
**Figure 57C** is a graphical representation of differential expression of SbPIN2 (Stg2 candidate) under well-watered conditions. Under well-watered conditions, this gene is slightly up-regulated in stem and root tissues of Tx642 and Stg1 NIL compared to Tx7000.
**Figure 57D** is a graphical representation of a differential expression of SbPIN2 (Stg 2 candidate) under water-deficient conditions. Under water-deficient conditions, this gene is up-regulated in most tissues of Tx642 and Stg1 NIL compared to Tx7000.

### DETAILED DESCRIPTION

Throughout this specification, unless the context requires otherwise, the word "comprise", or variations such as "comprises" or "comprising", will be understood to imply the inclusion of a stated element or integer or method step or group of elements or integers or method steps but not the exclusion of any other element or integer or method step or group of elements or integers or method steps.

As used in the subject specification, the singular forms "a", "an" and "the" include plural aspects unless the context clearly dictates otherwise. Thus, for example, reference to "a locus" includes a single locus, as well as two or more loci; reference to "an auxin" includes a single auxin, as well as two or more auxins; reference to "the disclosure" includes a single and multiple aspects taught by the disclosure.

The present disclosure teaches QTLs associated with and which facilitate the stay-green phenotype in crops including cereal plants. The QTLs are referred to generically as StgX wherein *X* is a numeral from 1 and above corresponding to a genetic locus or genetic loci region on a particular chromosome in a crop plant. A sub-region is referred to as StgXm where m is an alphabectical designation of a region within StgX. Modulation of expression of an StgX in all or selected tissue in a plant is taught herein to facilitate a physiological and genetic network which induces or promotes a shift in water use by the crop plant to the post-anthesis period or increased accessibility of water during crop growth or increased transpiration efficiency, thereby increasing harvest index (HI) and grain yield under water-limited conditions. "Expression" of an StgX includes up-regulating or down-regulating expression levels (i.e. modulation of expression) of a locus as well as selection of a polymorphic variant which is expressed at a higher or lower level or which encodes a more or less active product in all or selected tissue in a plant. The locus may itself confer this phenotype or a functional equivalent thereof such as a cDNA encoding the same protein encoded by the locus.

The QTLs identify loci encoding PIN proteins. PIN proteins are auxin efflux carriers which contain transmembrane domains and are mainly localized in the plasma membranes. The term "PIN" is derived from "pin-like inflorescence". A PIN from sorghum is an "SbPIN". The instant disclosure discloses use of PINs from any plant (e.g. an OsPIN from rice). The genomic locations of SbPINs from sorghum are described in Table 1A.

Provided herein is a method for generating a genetically modified plant which uses water more efficiently than a non-genetically modified plant of the same species, the method comprising introducing by genetic engineering using recombinant means genetic material which encodes an SbPIN4 protein to confer a stay-green phenotype, which phenotype includes a shift in water use to the post-anthesis period or increased accessibility of water during crop growth or increased transpiration efficiency resulting in increased harvest index and grain yield under water-limited conditions, wherein said SbPIN4 protein is encoded by Sorghum Gene ID Sb03g037350 from bp 65310051 to bp 65313194.

The methods disclosed herein for generating a genetically modified plant which uses water more efficiently than a non-genetically modified plant of the same species more generally includes introducing into a plant or parent of the plant a genetic agent which encodes a sorghum SbPIN protein selected from SBPIN1 through SbPIN11 or an equivalent in another plant or which modulate expression of an indigenous PIN. Examples of PINs include SbPIN4 and SbPIN2 and other SbPINs listed in Table 1A and their equivalents in another plant as well as a PIN having a particularly desired polymorphic variation which, for example, permits an altered expression profile of elevated levels of the PIN protein. Examples of PINs in other plants OsPIN5 which corresponds to SbPIN4 and OsPIN3a which corresponds to SbPIN2.

The genetic agent may be a locus or genomic region or its functional equivalent such as cDNA or a genomic DNA fragment. The agent may modulate expression of an indigenous PIN locus in a particular plant, but as claimed herein the agent encodes an SbPIN4 protein. By "introducing" means by recombinant intervention, or by mutagenesis or breeding followed by selection, but as claimed herein the agent is introduced by genetic engineering using recombinant means.

Without intending to limit the technology of the present specification to any one theory or mode of action, modulation of expression of a PIN alone or in combination with a genetic or physiological network alters plant architecture to enhance or otherwise promote efficient water use. In one aspect, the modified architecture is modified plant canopy architecture.

The term "progeny" includes immediate progeny as well as distant relatives of the plant, as long as it stably expresses the stay-green trait first introduced to an earlier parent.

Reference to a "crop plant" includes a cereal plant. The crop plants enabled herein include sorghum, wheat, oats, maize, barley, rye, rice, abaca, alfalfa, almond, apple, asparagus, banana, bean-phaseolus, blackberry, broad bean, canola, cashew, cassava, chick pea, citrus, coconut, coffee, corn, cotton, fig, flax, grapes, groundnut, hemp, kenaf, lavender, mano, mushroom, olive, onion, pea, peanut, pear, pearl millet, potato, ramie, rapeseed, ryegrass, soybean, strawberry, sugarbeet, sugarcane, sunflower, sweetpotato, taro, tea, tobacco, tomato, triticale, truffle and yam. In an example, the drought tolerance mechanisms of sorghum are used to promote drought tolerance in sorghum as well as other crop plants. A genetically modified plant generated by the method of the invention uses water more efficiently than a non-genetically modified plant of the same species. Existing PIN loci in each of the above plants are referred to as "indigenous" PINs. The instant disclosure teaches up- and down-regulating an indigenous PIN or selecting a PIN having a particular expression profile. An "indigenous" PIN is a PIN locus in a parent plant prior to any manipulation (recombinant, mutagenisis or breeding).

By "drought tolerance" includes drought escape, drought adaptation, drought resistance, reduced sensitivity to drought conditions, enhanced water use efficiency as well as an ability to shift water use to the post-anthesis period or increased accessibility of water during crop growth, thereby increasing HI and grain yield under water-limited conditions. Plants exhibiting drought tolerance are described as "drought adapted plants" or "plants exhibiting reduced sensitivity to water-limited conditions". Taught herein is that drought tolerance is induced, facilitated by or otherwise associated with the stay-green phenotype.

By "genetically modified", in relation to a plant, includes an originally derived genetically modified plant as well as any progeny, immediate or distant which stably express the stay-green trait. The present disclosure teaches the use of genetic engineering technology to introduce the genetic agent. This is encompassed by the terms "genetic engineering means" and "recombinant means". Markers defining stay-green can also be screened during breeding protocols to monitor transfer of particular genetic regions. Furthermore, a specific stay-green region is genetically inserted by recombinant means into a plant cell or plant callus and a plantlet regenerated. A "genetically modified" plant includes a parent or any progeny as well as any products of the plant such as grain, seed, propagating material, pollen and ova. In addition, a PIN locus may be expressed in one particular plant tissue but not expressed or its expression reduced in another tissue. Furthermore, a plant may be subject to mutagenisis such as genetic, radioactive or chemical mutagenisis and mutated plants selected with a PIN having a desired phenotype.

Reference to the "stay-green phenotype" includes characteristics selected from enhanced canopy architecture plasticity, reduced canopy size, enhanced biomass per unit leaf area at anthesis, higher transpiration efficiency, increased water use during grain filling, increased plant water status during grain filling, reduced pre:post anthesis biomass ratio, delayed senescence, increased grain yield, larger grain size, and reduced lodging.

Hence, taught herein is the use of genetic material encoding an SbPIN4 to facilitate the stay-green phenotype.

Enabled herein are genetically modified plants which exhibit the stay-green phenotype as a result of the genetic modification as well as seeds, fruit, flowers and other reproductive or other propagating material. Also enabled are root stock and propagating stock. This is based on the premise that the seeds, fruit, flowers, reproductive and propagating material exhibit or can pass on the stay-green phenotype introduced into the ultimate parent(s).

Reference to an "agent which modulates levels of expression of a PIN" includes promoters, microRNAs, genes and chemical compounds which facilitate increased or decreased expression of the gene in all or selected tissue or increased or decreased activity of a gene product as well as cDNA and genomic fragments. An agent may also be an intron of a genomic gene which is part of a natural genetic network to facilitate modulation of expression.

A PIN protein produces an auxin gradient in cells and contains a transmembrane domain and is mainly localized in the plasma membrane. PIN proteins are the rate-limiting factors of auxin transport and provide vectorial direction for the auxin flows. It is taught herein that at least one of Stg1 or Stg2 encodes a PIN protein. Introduction of Stg1 or Stg2 de novo in a plant or elevation of its modulation or expression of an indigenous Stg1 or Stg2 is taught to facilitate exhibition of one or more features or sub-features associated with the stay-green phenotype.

As indicated above, PIN proteins are efflux carriers of auxin which mediate polar auxin transport (PAT) from cell to cell as opposed to the transport of auxin through the xylem (Rashotte et al., Plant Cell 13:1683-1697, 2000; Friml et al., Current Opinion in Plant Biology 6:7-12, 2003). The term 'PIN' is derived from the pin-like inflorescence which develop in Arabidopsis when auxin transport is defective.

It is taught by the present disclosure that sorghum SbPIN4 and SbPIN2 are major drought adaptation genes along with other SbPINs as well as their equivalents in other plants. Differences in auxin signalling explain all of the multiple phenotypes observed in plants with a PIN expression profile. Phenotypes exhibited by SbPIN4 or SbPIN2 plants for example are explained by changes in auxin efflux and include reduced tillering, smaller leaves (both length and width), reduced leaf mass and increased root:shoot ratio. Phenotypes exhibited by SbPIN4 or SbPIN2 plants for example can also be explained indirectly (or as emergent consequences of these direct effects) and include increased availability of water at anthesis, higher leaf N concentration at anthesis, increased transpiration and biomass per unit leaf area, higher transpiration efficiency, retention of green leaf area during grain filling, increased harvest index, higher grain yield, larger grain size and increased lodging resistance. Taught herein is that equivalents of SbPIN4 and SbPIN2 and other SbPINs are operative across other major cereal and crop species to enhance drought adaptation in localities worldwide where water limits crop growth post-anthesis.

In accordance with the teachings of the present specification, modulation of expression of a PIN selected from SbPIN1 to 11 such as SbPIN4 (Stg1) and SbPIN2 (Stg2) or their equivalent in another plant in all or selected tissue confers drought adaptation both directly, and indirectly, ultimately leading to higher grain yield, larger grain size, and lodging resistance under water-limited conditions.

The stay-green phenotype may involve the presence of multiple proteins such as two or more of SbPIN1 to 11 such as SbPIN4 and SbPIN2.

Taught herein is a method for generating a genetically modified plant which uses water more efficiently than a non-genetically modified plant of the same species, the method comprising introducing into a plant or parent of the plant a genetic agent which encodes two or more PINs including SbPIN4 to confer a stay-green phenotype, which phenotype includes a shift in water use to the post-anthesis period or increased accessibility of water during crop growth or increased transpiration efficiency resulting in increased harvest index and grain yield under water limiting conditions.

. By "two or more" means 2 or 3 or 4 or 5 or 6 or 7 or 8 or 9 or 10 or 11.

In an example, the genetic material encodes (i) SbPIN4 and (ii) an agent which modulates levels of expression of SbPIN4 or its equivalent in another plant, an agent which encodes SbPIN2 or an agent which modulates expression of SbPIN2 or its equivalent in another plant.

Increased water availability at anthesis is achieved via reduced water use due to two mechanisms (reduced tillering and smaller leaves) in plants containing the Stgl region. Both mechanisms, individually, appear to reduce canopy size by about 9%, on average. The 'low-tillering' mechanism dominates in low density environments when tillering potential is high. The 'small-leaf' mechanism dominates in high density environments when tillering potential is low. Combined, these two mechanisms provide crop plants with considerable plasticity to modify canopy architecture in response to the severity of water limitation.

Stay-green enhances canopy architecture plasticity via constitutive and adaptive responses. Canopy size in Stgl or Stg2 is reduced by about 5%, even when water is not limiting (constitutive response). Canopy size is further reduced (adaptive response) in a mild drought (∼10%) and more severe drought (∼15%). Low tillering is primarily a constitutive response. Small leaf size is both a constitutive and adaptive response.

Furthermore, it is taught herein that the Stgl or Stg2 region confers drought adaptation by reducing canopy size (via reduced tillering and smaller leaves) and reducing crop water use at anthesis. This is shown by a high correlation (r2=0.9) between canopy size and crop water use in artificial dought (rain-out shelter [ROS]) and lysimeter studies.

Increased water availability at anthesis is also achieved via increased water accessibility (better water extraction and deeper or greater lateral spread).

Stay-green enhances biomass per unit leaf area at anthesis. Assuming root mass is equivalent (or at least not significantly less), these differences could be explained by differences in transpiration (T) per unit leaf area [LA] (T/LA) and/or transpiration efficiency (TE). Lysimetry studies indicate that increases in T/LA, rather than TE, drive the observed increases in biomass per leaf area. Note that increased T/LA only occurred when water deficit was sufficient to reduce leaf area. When water deficit was less severe (i.e. not enough to reduce leaf area), then T/LA decreased, resulting in higher TE.

Higher TE in StgX lines such as Stgl or Stg2 lines is also observed when water deficit is less severe. Increased TE via introgressing Stg1 or Stg2 is proposed to be due to a) proportionally higher photosynthetic capacity compared with stomatal conductance, due to smaller, thinner and greener leaves, and/or b) a decrease in transpiration while maintaining biomass. Lysimetry studies indicate that both of these mechanisms contribute to higher TE in Stgl lines, with the reduction in transpiration the primary mechanism.

Changes in transpiration per unit leaf area is taught herein to be due to a) number of stomata, b) size of stomatal aperture, c) changes in the timing of stomatal opening and closing relative to VPD, and/or d) the number of hair base cells (which affects the boundary layer and hence T/LA). Introgressing Stg1, for example, into RTx7000 modified leaf anatomy by increasing the number of bundle sheath cells surrounding the vascular bundle.

Differences in the morphology of leaves are apparent between RTx7000 and Stg1 or Stg2. In this case, there were more and smaller bundle sheaths surrounding the vascular bundle in Stg1 or Stg2. Also, there were fewer stomata and more hair base cells per unit leaf area (leaves 7 and 10) in Stg1 compared with RTx7000.

Increased water use during grain filling is achieved via (i) increased water availability at anthesis and (ii) increased water accessibility (better water extraction and deeper or greater lateral spread) during grain filling.

Crop water use (CWU) before anthesis was negatively correlated with CWU after anthesis in an artificial dought (rain-out shelter [ROS]) experiment. For example in one experiment, a 25% increase in water use after anthesis (80 vs 60 mm) resulted in a 25% increase in grain yield (400 vs 300 g/m2). This translated to 50 kg/ha of grain for every additional mm of water available.

Increased water use during the grain filling period was exhibited by Stg1 or Stg2 under both low and high density treatments in a rain-out shelter (ROS) experiment. This was due primarily to (i) reduced water use at anthesis under high density, and (ii) increased water accessibility during grain filling under low density.

It is taught herein that StgX such as Stg1 or Stg2 confers drought adaptation by being associated with pre- and post-anthesis biomass production. The Stg1 or Stg2 region, for example, reduces the pre:post anthesis biomass ratio below a critical level, increasing grain yield and lodging resistance.

In accordance with teachings of the the present disclosure, expression of StgX such as Stg1, Stg2, Stg3 and/or Stg4 facilitates one or more of the following phenotypes:
(i) delayed leaf senescence (stay-green), higher grain yield and lodging resistance are consequences of higher plant water status during grain filling (due to increased water use during grain filling);
(ii) introgressing StgX into a, for example, RTx7000 background increases plant water status at mid-grain filling, as indicated by a) higher relative water content (RWC), and b) lower leaf water potential (LWP);
(iii) higher grain yield and larger grain size are consequences of increased water availability during grain filling;
(iv) higher grain yield, larger grain size and increased lodging resistance are not mutually exclusive (i.e. all three traits are exhibited by StgX);
(v) yield and grain size advantage are relatively higher under severe terminal drought than mild terminal drought;
(vi) the benefit of the stay-green genes in a, for example, RTx7000 background (inbreds) occurs in the yield range of 1-3 t/ha (12-22%), followed by a lesser but still significant benefit in the 3-4 t/ha yield range (8-10%). There was, however, a small penalty associated with these regions (2-4%) at higher yield levels (5-8 t/ha) due to wetter conditions. Note that these yield ranges would be considerably higher in hybrids. Since the average sorghum grain yield for hybrids in the northern grain belt is about 2.5 t/ha, the benefit of the stay-green genes should be significant. No reduction in grain yield under wetter conditions (water not limiting) due to stay-green has been observed in hybrids;
(vii) introgressing StgX into, for example, RTx7000 also increases grain size by 11%, on average, under severe terminal drought. There was no impact of the StgX QTL on grain size under a mild terminal drought or under no drought; and
(viii) each of the key StgX mechanisms maps to a defined region, suggesting that the action of a single gene has multiple pleiotrophic effects.

Also disclosed but not claimed herein is a business model to enhance economic returns from crop production. According to these teachings, provided is a business model for improved economic returns on crop yield, the model comprising generating crop plants having a PIN expression profile resulting in the crop plant having a shift in water use by the plant to the post-anthesis period or increased accessibility of water during crop growth thereby increasing HI and grain yield under water-limited conditions, obtaining seed from the generated crop plant and distributing the seed to grain producers to yield enhanced yield and profit. Reference to a PIN includes SbPIN1 to 11 such as SbPIN4 and SbPIN2, as well as their equivalents in other plants.

Further disclosed herein is a plant management system to reduce crop reliance on water or to otherwise improve water use efficiency and to enhance grain or product yield. The plant management system includes the generation of drought adapted crop including cereal plants using the selection and modulated expression of PIN locus or its functional equivalent as herein defined alone or in combination with the introduction of other useful traits such as grain size, root size, salt tolerance, herbicide resistance, pest resistance and the like. Alternatively or in addition, the plant management system comprises generation of drought adapted plants and agricultural procedures such as irrigation, nutrient requirements, crop density and geometry, weed control, insect control, soil aeration, reduced tillage, raised beds and the like.

The present specification is instructional as to a means to induce or enhance drought adaptation capacity in a plant by introducing do novo one or more features of the stay-green phenotype or elevating or reducing expression of an existing one or more PIN loci in a plant and/or selecting an PIN polymorphic variant with improved or enhanced expression or product activity. The manipulation of the stay-green phenotype may be done alone or as part of an integrated plant management system which may include further trait selection and/or improved agronomic techniques. The resulting crops use water more efficiently and have a higher yield of grain and increased grain size.

The present disclosure is enabling in the respect of the use of a genetic agent encoding a PIN protein in the manufacture of a drought-adapted plant.

QTLs are identified herein as carrying one or more PIN loci wherein the level of expression of which is selected by genetic engineering, to promote a stay-green phenotype.

Genetically modified plants and their progeny exhibiting the stay-green trait are also disclosed herein as well as seeds, fruit and flowers and other reproductive or propagating material.

Genetic material which encodes a PIN protein or functional homolog or ortholog thereof which is associated with or facilitates a stay-green phenotype, which phenotype includes a canopy architecture which facilitates a shift in water use to the post-anthesis period or increased accessibility of water during crop growth or increased transpiration efficiency resulting in increased harvest index and grain yield under water limiting conditions; and an agent which modulates the level of the PIN; are both disclosed herein.

In the invention, the genetic material includes an agent which encodes SbPIN4; and optionally an agent which encodes SbPIN2. However, an agent may also modulate levels of expression of SbPIN4 or SbPIN2 or an equivalent thereof in another plant.

The genetic material includes an agent which encodes SbPIN4, which confers a stay-green phenotype, which phenotype includes a canopy architecture which facilitates a shift in water use to the post-anthesis period or increased accessibility of water during crop growth or increased transpiration efficiency resulting in increased harvest index and grain yield under water limiting conditions. The subject genes may also be used as markers to transfer regions of genomes comprising one or more of the genes, or their equivalents in other plants, to a particular plant in order to include the stay-green phenotype.

### EXAMPLES

Aspects taught and enabled herein are further described by the following non-limiting Examples.

### EXAMPLE 1

### Identification of an StgX gene

A quantitative trait locus (QTL), which is referred to herein as Stg1 which is an example of an StgX, has been identified which increases or enhances water use efficiency by sorghum plants. Stg1 encodes a sorghum bicolor member of the auxin efflux carrier component 4 family, PIN4 (or SbPIN4).

This major drought adaptation gene has been fine-mapped on the sorghum genome. Changes in auxin efflux could explain all of the multiple phenotypes observed in plants containing SbPIN4. The candidate gene (and promoter region) is sequenced in the two parents of the fine-mapping population (RTx7000 and Tx642) to identify a single nucleotide polymorphism. RNA expression profiling of the Stg1 fine-mapping population is also conducted for a subset of lines, times and organs. Phenotypes exhibited by SbPIN4 plants that could be explained directly by changes in auxin efflux include reduced tillering, smaller leaves (both length and width), reduced leaf mass and increased root:shoot ratio. Phenotypes exhibited by SbPIN4 plants that could be explained indirectly (or as emergent consequences of these direct effects) include increased availability of water at anthesis, higher leaf N concentration at anthesis, increased transpiration and biomass per unit leaf area, reduced pre:post anthesis biomass ratio, higher transpiration efficiency, retention of green leaf area during grain filling, increased harvest index, higher grain yield, larger grain size and increased lodging resistance. It is proposed that SbPIN4 works across other major cereal and crop species to enhance drought adaptation in localities worldwide where water limits crop growth post-anthesis.

Stg1 (SbPIN4) confers drought adaptation both directly, and indirectly, ultimately leading to higher grain yield, larger grain size, and lodging resistance under water-limited conditions.

Increased water availability at anthesis is achieved via reduced water use due to two mechanisms (reduced tillering and smaller leaves) in plants containing the Stg1 region. Both mechanisms, individually, appear to reduce canopy size by about 9%, on average. The 'low-tillering' mechanism dominates in low density environments when tillering potential is high. The 'small-leaf' mechanism dominates in high density environments when tillering potential is low. Combined, these two mechanisms provide crop plants with considerable plasticity to modify canopy architecture in response to the severity of water limitation.

Stay-green enhances canopy architecture plasticity via constitutive and adaptive responses. Canopy size in Stgl is reduced by about 5%, even when water is not limiting (constitutive response). Canopy size is further reduced (adaptive response) in a mild drought (∼10%) and more severe drought (∼15%). Low tillering is primarily a constitutive response. Small leaf size is both a constitutive and adaptive response.

There is a link between reduced canopy size (via reduced tillering and smaller leaves) and reduced crop water use at anthesis. High correlation (r2=0.9) between canopy size and crop water use in ROS and lysimeter studies.

Increased water availability at anthesis is also achieved via increased water accessibility (better water extraction and deeper or greater lateral spread).

Stay-green enhances biomass per unit leaf area at anthesis. Assuming root mass is equivalent (or at least not significantly less), these differences could be explained by differences in transpiration per unit leaf area (T/LA) and/or transpiration efficiency (TE). Lysimetry studies indicate that increases in T/LA, rather than TE, drive the observed increases in biomass per leaf area. Note that increased T/LA only occurred under low VPD conditions; T/LA was actually reduced under high VPD conditions, presumably as a water conservation mechanism.

Higher TE in Stg1 lines was also observed under higher VPD conditions. Increased TE via introgressing Stg1 may be due to a) proportionally higher photosynthetic capacity compared with stomatal conductance, due to smaller, thinner and greener leaves, and/or b) a decrease in transpiration while maintaining biomass. Lysimetry studies indicate that both of these mechanisms contribute to higher TE in Stgl lines, with the reduction in transpiration the primary mechanism.

Changes in transpiration per unit leaf area could be due to a) number of stomata, b) size of stomatal aperture, c) changes in the timing of stomatal opening and closing relative to VPD, and/or d) the number of hair base cells (which affects the boundary layer and hence T/LA). Introgressing Stgl into RTx7000 reduced the number of stomata and increased the number of hair base cells per unit leaf area in leaves 7 and 10; both mechanisms can conserve water by reducing T/LA.

Introgressing Stgl into RTx7000 modified leaf anatomy by increasing the number of bundle sheath cells surrounding the vascular bundle. The increased number of cells in the bundle sheath might also contribute to increased photosynthetic assimilation and hence TE.

Differences in the morphology of leaves (e.g. Leaves 7 and 10) are apparent between Tx7000 and Stg1. In this case, there were more and smaller bundle sheaths surrounding the vascular bundle in Stg1. The increased number of cells in the bundle sheath might also contribute to increased photosynthetic assimilation and hence TE.

Increased water use during grain filling is achieved via (i) increased water availability at anthesis and (ii) increased water accessibility (better water extraction and deeper or greater lateral spread) during grain filling.

### a) Increased water availability at anthesis

Crop water use (CWU) before anthesis was negatively correlated with CWU after anthesis in an ROS experiment. For example, in one experiment a 25% increase in water use after anthesis (80 vs 60 mm) resulted in a 25% increase in grain yield (400 vs 300 g/m2). This translated to 50 kg/ha of grain for every additional mm of water available.

### b) Increased water accessibility during grain filling

Increased water use during the grain filling period was exhibited by Stgl under both low and high density treatments in an ROS experiment. This was due primarily to (i) reduced water use at anthesis under high density, and (ii) increased water accessibility during grain filling under low density.

*Stg1* region confers drought adaptation via a link between pre- and post-anthesis biomass production. The Stgl region reduces the pre:post anthesis biomass ratio below a critical level, increasing grain yield and lodging resistance.

Delayed leaf senescence (stay-green), higher grain yield and lodging resistance are consequences of higher plant water status during grain filling (due to increased water use during grain filling).

Introgressing Stg1 into a RTx7000 background increased plant water status at mid-grain filling, as indicated by a) higher relative water content (RWC), and b) lower leaf water potential (LWP).

Higher grain yield and larger grain size are consequences of increased water availability during grain filling.

Higher grain yield, larger grain size and increased lodging resistance are not mutually exclusive (i.e. all three traits are exhibited by Stg1).

Yield and grain size advantage are relatively higher under severe terminal drought than mild terminal drought.

Studies indicate that the greatest benefit of the stay-green genes in a RTx7000 background (inbreds) occurs in the yield range of 1-3 t/ha (12-22%), followed by a lesser but still significant benefit in the 3-4 t/ha yield range (8-10%). There was, however, a small penalty associated with these regions (2-4%) at higher yield levels (5-8 t/ha) due to wetter conditions. Note that these yield ranges would be considerably higher in hybrids. Since the average sorghum grain yield for hybrids in the northern grain belt is about 2.5 t/ha, the benefit of the stay-green genes should be significant. Overall, no reduction in grain yield under wetter conditions (water not limiting) due to stay-green has been observed in hybrids.

Introgressing Stg1 into RTx7000 also increased grain size by 11%, on average, under severe terminal drought. There was no impact of this QTL on grain size under a mild terminal drought or under no drought.

Each of the key Stg1 mechanisms maps to the same region, indicating the action of a single gene with multiple pleiotrophic effects.

### EXAMPLE 2

### Reduced tillering (physiological studies of NILs in the field)

Data show the impact of Stg1 on tillering under both high water (HW) and low water (LW) conditions. Differences in canopy development before flowering were largely a consequence of variation in tillering among lines. Culm number per m2 at anthesis was the best overall measure of the effect of tillering on canopy dynamics. Culm numbers per m2 were equivalent under both water regimes (12.89), indicating that reduced tillering is a constitutive trait. Genotypes varied significantly (P<0.001) in this parameter, ranging from 8.59 to 16.67. However, genotype and treatment did not interact significantly for this parameter.

Culm numbers per m2 were analyzed in terms of their Stg status and category means are presented in Table 1. RTx7000 produced 41% more (P<0.05) culms/m2 than B35 (14.07 vs. 10.00). Introgression of the Stg1 region alone into RTx7000 (6078-1) reduced culms/m2 significantly (P<0.05) compared with RTx7000 (9.40 vs. 14.07). Compared with Stg1 only, additional introgressions of either Stg2 or Stg4 increased culm numbers to 10.49 (1,2 combination) and 10.74 (1,4 combination). Note that the three near-isolines containing no Stg regions (2212-3, 2235-11 and 6120-16) also exhibited high tillering equivalent to Tx7000. Hence the overall ranking of tillering in these lines is Stg1 < B35 < Stg4 < Stg2 < Stg3 < none < RTx7000.

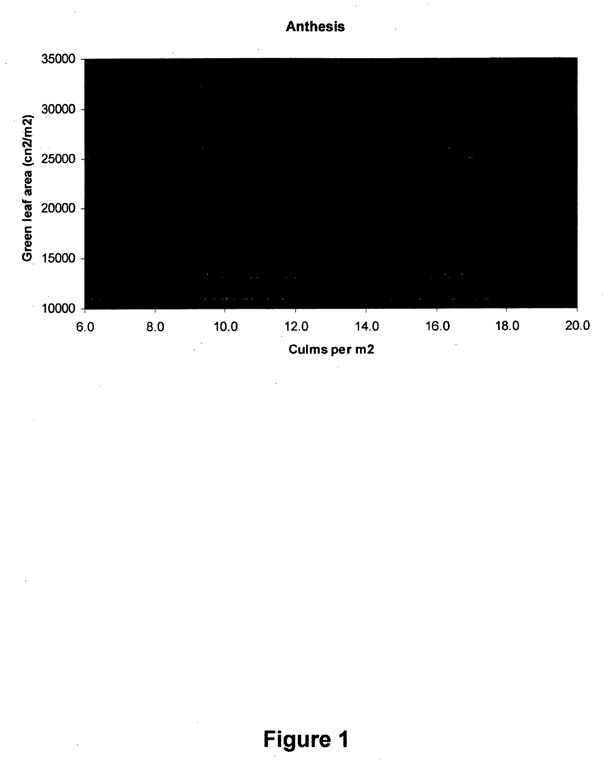

At anthesis, culm numbers were highly correlated (r2=0.71) with total green leaf area (GLAA; Figure 1).

**Table 1**

| *Stg* status | No of lines | Culms/m2 A |
|---|---|---|
| | | |

| *Stg1 region* | | |
|---|---|---|
| 1 | 1 | 9.40 |
| 1,2 | 2 | 10.49 |
| 1,4 | 1 | 10.74 |
| | | |
| RTx7000 | 7 | 14.07 |
| | | |
| LSD (0.05) | | 4.06 |

Culms per m2 in the recurrent parent (RTx7000) and various lines containing introgressions of the Stg1 QTL, both alone and in combination with other Stg QTL.

Differences in green leaf area at anthesis (GLAA) were primarily due to differences in tiller green leaf area at anthesis (GLAAt), since GLAAt was highly correlated with GLAA (r²=0,78), yet mainstem leaf area was not.

Tiller green leaf area at anthesis was analyzed in terms of Stg status and category means are presented in Table 2. RTx7000 produced almost eight-fold more (P<0.05) GLAAt than B35 (15460 vs. 1980). Introgression of the Stgl region alone into RTx7000 (6078-1) reduced GLAAt significantly (P<0.05) compared with RTx7000 (3121 vs 15460). Compared with Stg1 only, additional introgressions of either Stg2 or Stg4 increased GLAAt to 4187 (1,2 combination) and 4797 (1,4 combination). All lines containing Stg1 (in any combination) were not significantly different (P<0.05) in GLAAt from Stg1 alone. Note that GLAAt in the three near-isolines containing no Stg regions (2212-3, 2235-11 and 6120-16) was not significantly different from RTx7000. Hence the significantly different (P<0.05) rankings of GLAAt in these lines are B35 = *Stg1* = *Stg4* < *Stg2* = *Stg3* < none = RTx7000.

**Table 2**

| *Stg* status | No of lines | Tiller green leaf |
|---|---|---|
| | | area at anthesis |
| | | (cm2/m2) |

| *Stg1 region* | | |
|---|---|---|
| 1 | 1 | 3121 |
| 1,2 | 2 | 4187 |
| 1,4 | 1 | 4797 |
| | | |
| RTx7000 | 7 | 15460 |
| | | |
| LSD (0.05) | | 5282 |

Tiller green leaf area at anthesis in the recurrent parent (RTx7000) and various lines containing introgressions of the Stg1 QTL, both alone and in combination with other Stg QTL.

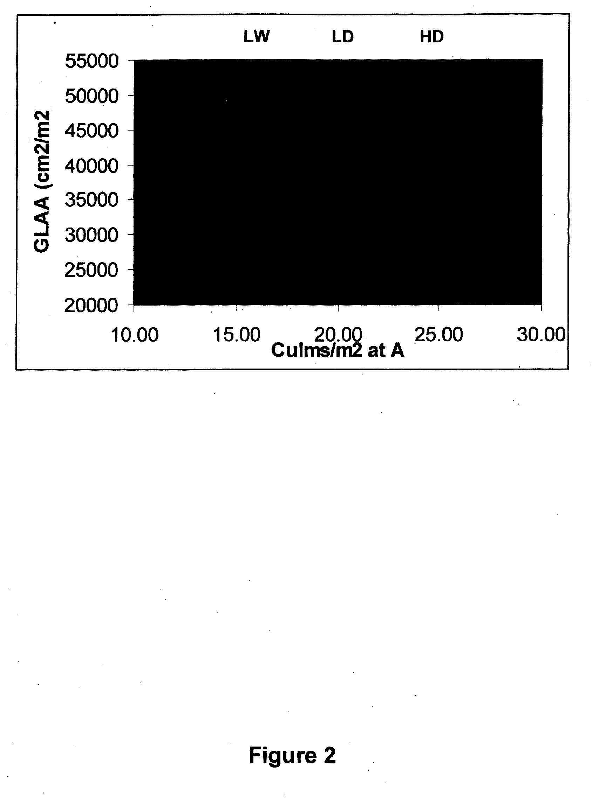

Green leaf area and culms per m2 at anthesis were highly correlated under high (HD) and low (LD) density treatments in the rainout shelter experiment (Figure 2). Introgressing the Stg1 or Stg1a regions into RTx7000 reduced culms per m2 and GLAA under both densities.

### EXAMPLE 3

### Reduced tillering (fine-mapping studies)

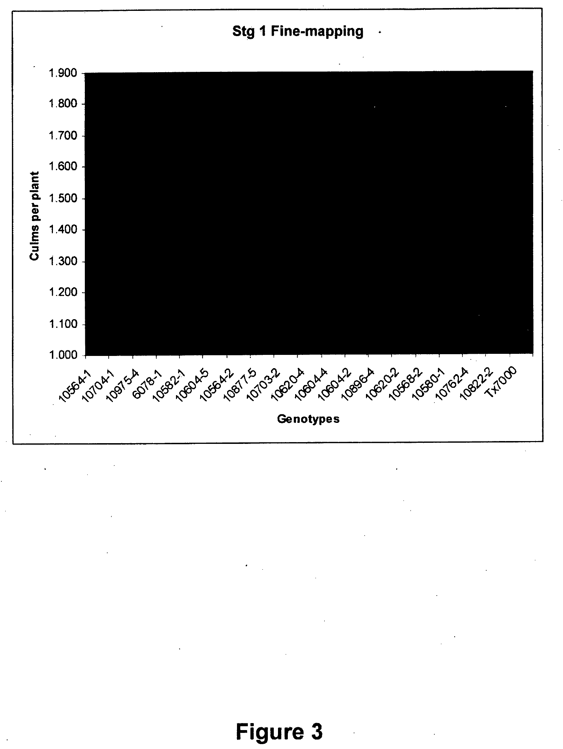

A Stg1 fine-mapping population was grown in the field under high and low density conditions in three consecutive years. The number of culms per plant was measured at anthesis in each year and a combined analysis was undertaken across years. Overall, RTx7000 produced 47% more culms per plant than 6078-1 (1.85 vs. 1.26; Figure 3).

In these field studies, the trait (culm number per plant) was mapped. An arbitrary value of 1.54 culms per plant gave the optimal separation between high and low tillering for mapping purposes (i.e. recombinants with less than 1.54 culms were BB genotypes while those with more than 1.54 culms were TT genotypes). Stepping down through the markers reveals that gain-of-function (low tillering) was achieved in three genotypes (10564-2, 10704-1 and 10620-4). One recombinant (10568-2) exhibited a high tillering phenotype, while three others (10620-4, 10704-1 and 10564-2) exhibited low tillering phenotypes. An auxin efflux carrier component 5 gene was proposed to be the candidate gene.

### EXAMPLE 4

### Reduced tillering (Stg1 fine-mapping studies in the ROS)

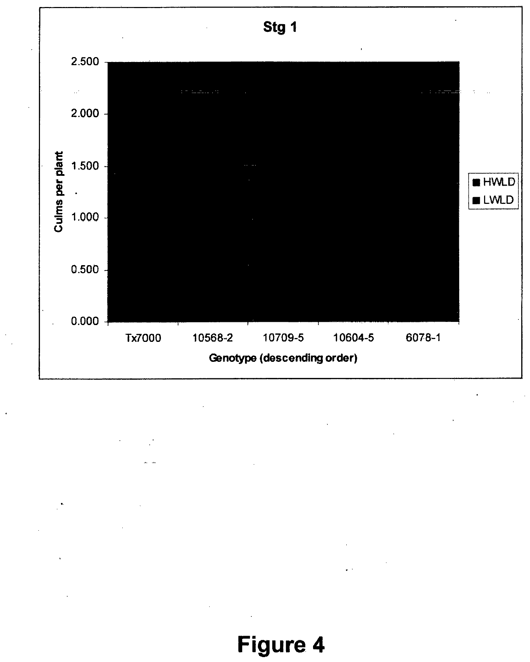

A subset of the Stgl fine-mapping population was grown in the field at the Rain-Out Shelter (ROS) under high and low water conditions, with each water treatment split for high and low density. This created four water regimes with increasing levels of water deficit: HWLD (least stressed) <HWHD<LWLD<LWHD (most stressed). The number of culms per plant was measured at 44 days after emergence in each plot. Differences were most obvious in the Low Density (LD) treatment, since expression of tillering is maximized in this treatment. On average, RTx7000, 10568-2 and 10709-5 produced 27% more culms per plant than 6078-1 and 10604-5 under LWLD conditions (2.05 vs. 1.62; Figure 4), and 23% more culms per plant under HWLD conditions (1.49 vs. 1.22).

### EXAMPLE 5

### Reduced tillering (Stg1 fine-mapping studies in the igloo)

Three additional fine-mapping studies were undertaken on the Stgl population under controlled conditions in an igloo. In these studies, tillering was analyzed in more detail compared with the earlier field studies. The total number of tillers was counted and, more specifically, the number of tillers emerged from the axil of leaves 2 (T2), 3 (T3) and 4 (T4) were counted. Presence or absence of T2 was the best indicator of overall tillering potential for a given recombinant. T2 was also the best trait to use for fine-mapping the gene.

In this experiment, the total number of tillers was the sum of T2, T3 and T4, where T2 was the tiller emerging from the axil of leaf 2 (and so on for T3 and T4), including secondary tillers. Significant genotypic variation was observed for all of the traits relating to tillering in this study (Table 34), with heritabilities generally above 30.

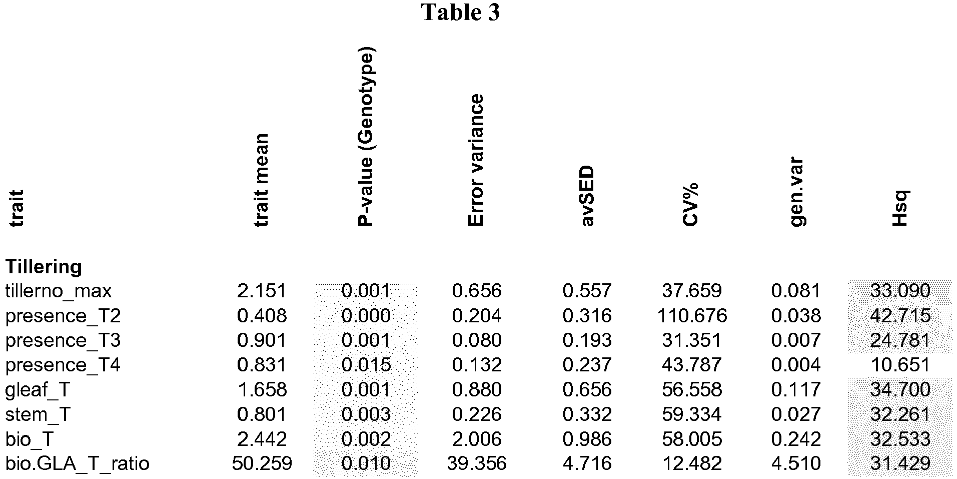

Table 3 provides a summary of predicted means, P-value and heritability of tillering traits measured at the L11 harvest. Significant differences (P<0.05) are shaded in yellow while heritabilities >20 are shaded in green.

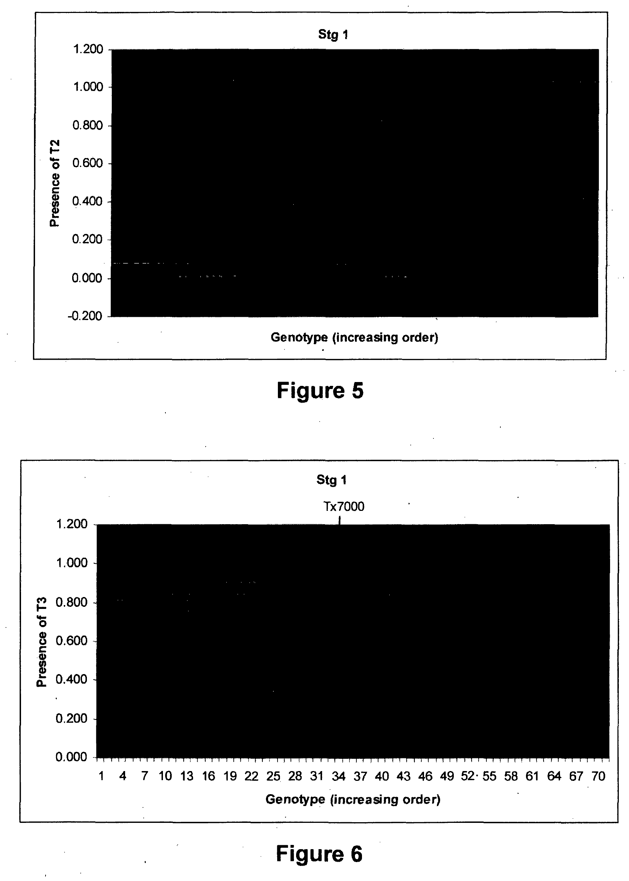

Separate analysis of the T2, T3 and T4 data found that 6078-1 produced no T2 tillers in any of the four replicates, while RTx7000 produced T2 tillers in 2 of 4 replicates (Figure 5).

Differences were also apparent in T3 numbers. 6078-1 produced a T3 tiller in 1 of 4 replicates, while RTx7000 produced a T3 tiller in all 4 replicates (Figure 6).

T4 tiller numbers also varied among genotypes. 6078-1 produced a T4 tiller in 3 of 4 replicates, while RTx7000 produced a T4 in all 4 replicates. Hence the Stg1 introgression essentially prevented the growth of T2 and T3 tillers in a RTx7000 background.

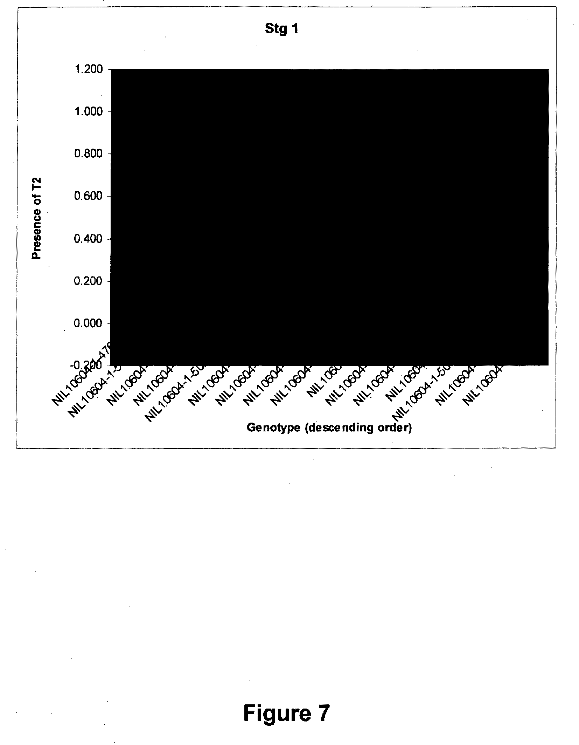

Lines were separated which produced a T2 tiller in 0-2 replicates (low tillering group; 8 recombinants) from those that produced a T2 tiller in 3-4 replicates (high tillering group; 8 recombinants) [Table 4, Figure 7].

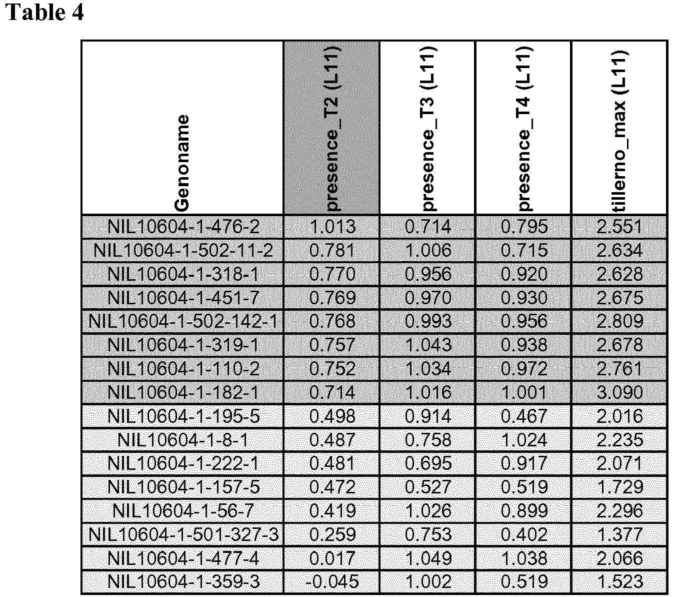

Table 4 shows the presence of tillers (T1-T3) and total tiller number for eight high-tillering recombinants (brown shading) and eight low-tillering recombinants (green shading) from the Stg1 fine-mapping population.

Gain-of-function (low tillering) is achieved in recombinant 10604-1-157-5. Gain-of-function (low tillering) is also achieved in three recombinants (10604-1-195-5, 10604-1-56-7, 10604-1-477-4).

A subset of lines was used in this experiment to validate the tillering region. More replicates per recombinant (20) were used to further reduce the error variance and increase the power of discrimination among lines. Results indicated the presence of an auxin efflux corner gene.

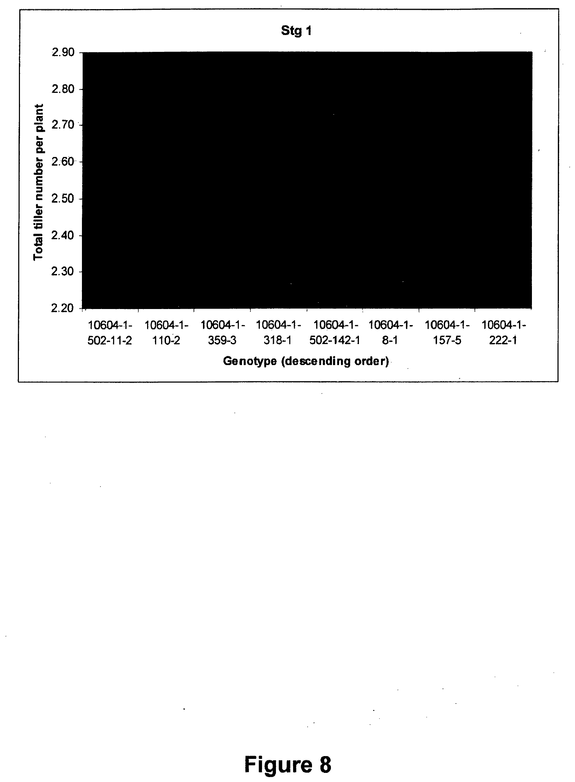

A breakpoint analysis of those lines which, according to their genotype (BB or TT), 'step up' or 'step down' through the region of interest, was undertaken to further pinpoint the low-tillering gene. A clear break was apparent, separating lines that produced a total tiller number >2.5 (high tillering group; 5 recombinants) from those that produced a total tiller number <2.5 (low tillering group; 3 recombinants) [Table 5, Figure 8].

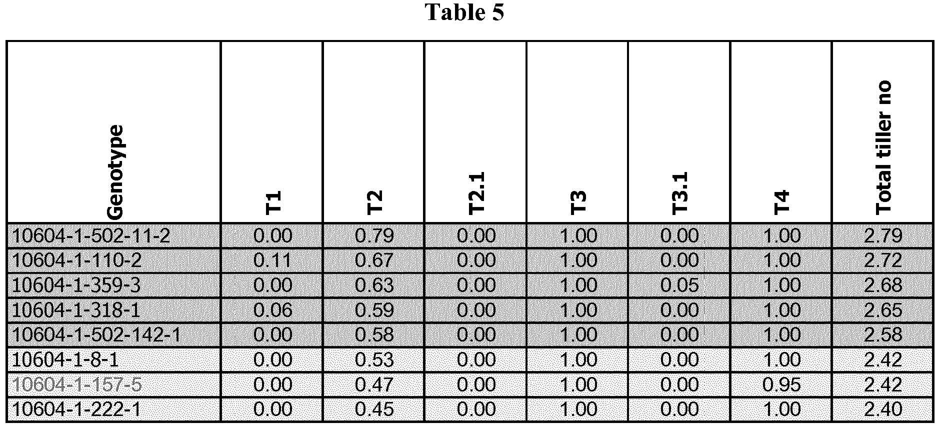

Table 5 shows the presence of tillers (T1-T4), including secondary tillers, and total tiller number for five high-tillering recombinants (brown shading) and three low-tillering recombinants (green shading) from the Stgl fine-mapping population.

Gain-of-function (low tillering) is achieved in recombinant 10604-1-157-5. The PIN4 gene in Sorghum bicolor (designated herein "SbPIN4") is therefore a strong candidate for the Stg1 low tillering gene.

### EXAMPLE 6

### Smaller leaves

Overall, introgressing the Stg1 region into RTx7000 reduced leaf size (length and width) under well-watered and water-limited conditions, indicating a constitutive gene action. However, the reduction in leaf size was generally greater under water-limited conditions indicating, to some extent, an adaptive (inducible) response in addition to the constitutive response. Hence Stg1 confers two mechanisms for reducing canopy size: a) reduced tillering, and b) reduced leaf size. Combined, these two mechanisms provide a fair degree of plasticity for the plant to modify canopy architecture in response to environmental and/or management factors.

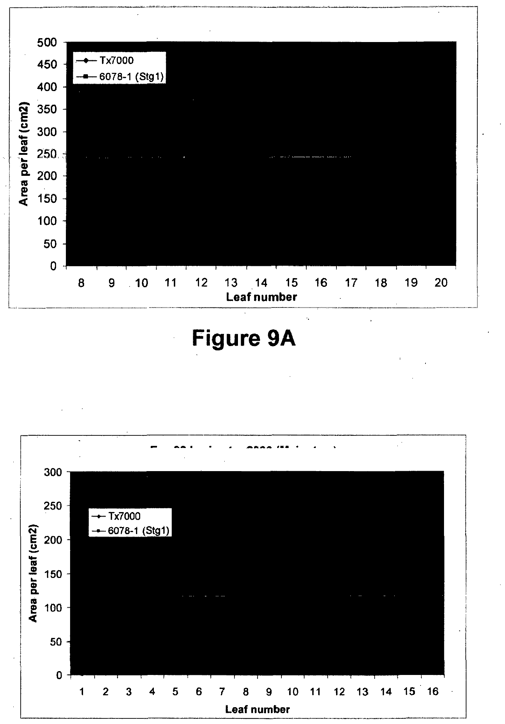

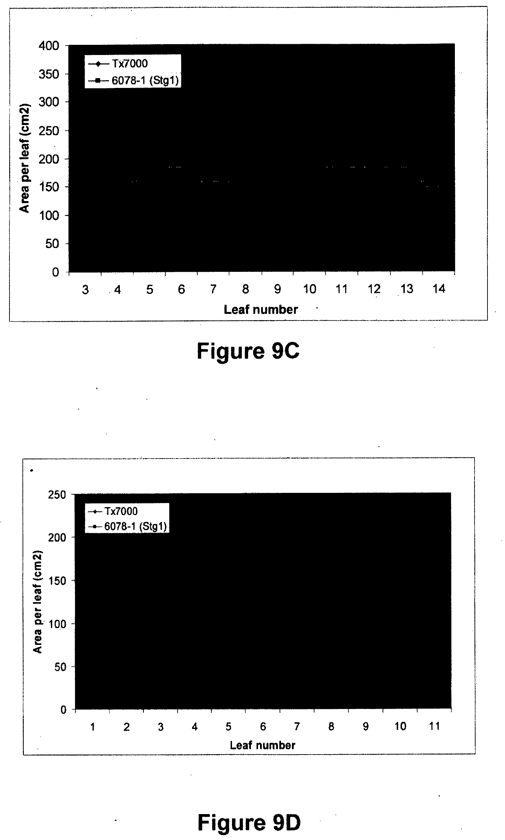

A series of lysimeter studies is particularly instructive in assessing leaf size patterns under varying levels of vapor pressure deficit (VPD) (Figures 9A through D). Although pots were regularly watered in both experiments, the canopy size differed between seasons, presumably due to seasonal differences in temperature and VPD, creating high (1.8 kPa) and low (1.3 kPa) VPD conditions. For the mainstem and the largest tiller (T3), the reduction in leaf size was significant under both high and low VPD conditions (Figures 9A through D), although the reduction commenced later under low VPD compared with high VPD in the mainstem (L12 vs L9) and T3 (L7 vs L5).

However for the remaining tillers (T4-T6), the leaf size distributions differed markedly between experiments. While there was no difference in leaf size between RTx7000 and 6078-1 under high VPD, the leaves of 6078-1 were significantly smaller under low VPD. This indicates an adaptive (inducible) response to leaf size reduction under certain environmental conditions for tillers T4-T6.

Hence, under low water stress, introgressing Stg1 into RTx7000 resulted in smaller leaves in the mainstem and largest tiller (T3), but not in T4-T6. This response would enable the plant to maximise light interception in the later tillers when conditions are favorable. Under high water stress, introgressing Stg1 into RTx7000 resulted in smaller leaves in all culms. This response would enable the plant to dramatically reduce its canopy size under water-limited conditions.

### EXAMPLE 7

### Smaller leaves (Rain-Out Shelter studies)

Experiments were conducted under the Rain-Out Shelter (ROS) to assess the impact of the Stg1 region under two crop densities, thereby creating two levels of water deficit (high density = high stress; low density = low stress). In general, tillering was low or absent under HD (20 plants/m2) and normal under LD (10 plants/m2).

Canopy size was smaller in both years under the high density (HD) treatment, reflecting the greater water deficit generated by this treatment. In both years under the milder (LD) and more severe (HD) water deficits, leaf sizes were generally smaller in 6078-1 (Stg1) compared with RTx7000. The exception was where the leaf size distribution pattern was similar for 6078-1 and RTx7000 in the milder water deficit (LD), yet leaves were significantly smaller in 6078-1 (up to 18% smaller) under greater water deficit (HD), suggesting an adaptive response by Stg1 plants to increasing water deficit. In fact, introgressing the Stgl region into RTx7000 reduced the size of the four largest leaves (L10-L13) by an average of 16.5% in the more severe water deficit (HD). Since there was little tillering in either genotype in this treatment, reduced leaf size in 6078-1 should have markedly decreased canopy size and hence crop water use (assuming similar transpiration per unit leaf area).

Note that the leaf size reduction mechanism associated with Stg1 appears to operate in both the presence (LD) and absence of tillering (HD), but appears to be best expressed under HD where uniculm and high water deficit conditions generally occur.

### EXAMPLE 8

### Smaller leaves (Stg1 fine-mapping studies at Rain-Out Shelter)

A subset of the Stg1 fine-mapping population was grown in the field at the Rain-Out Shelter (ROS) under high and low water conditions, with each water treatment split for high and low density. This created four water regimes with increasing levels of water deficit: HWLD (least stressed) <HWHD<LWLD<LWHD (most stressed). The area of each fully-expanded mainstem leaf was measured for all genotypes in all treatments.

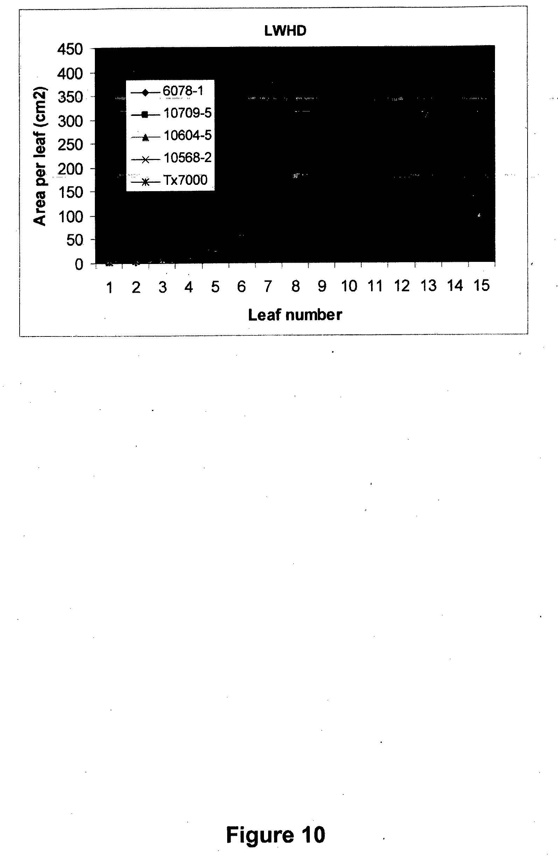

Introgressing the whole Stgl region (6078-1) and, more specifically, the smaller region designated 10604-5, resulted in a reduction in the size of leaves 9-13 under low-water and high-density conditions (Figure 10). For example compared with RTx7000, L11 was 9% and 16% smaller in 10604-5 and 6078-1, respectively. Since tillering was negligible in this treatment, differences in canopy size were essentially due to differences in leaf size.

Under low density conditions, leaf size distributions were affected by tillering, resulting in some crossovers compared with the high density treatment (see Figure 10). However, note that 10604-5 produced smaller leaves 9-13 relative to 10709-5, 10568-2 and RTx7000 in both HD and LD treatments.

The "small leaf size" gene mapped to the candidate gene, auxin efflux carrier component 5.

### EXAMPLE 9

### Smaller leaves (fine-mapping studies in igloo)

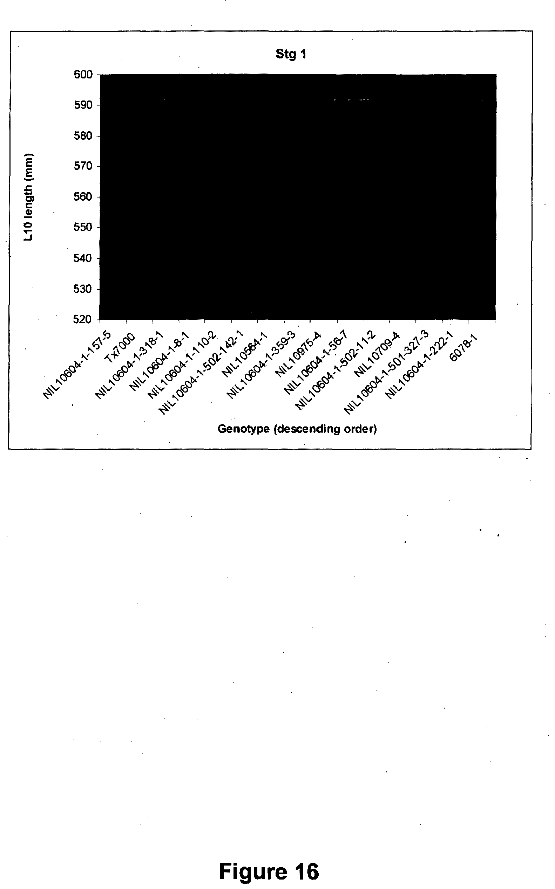

Leaf area varied significantly (P<0.001) among genotypes with a heritability approaching 60 for leaves 4 and 5. Introgressing the Stg1 region into RTx7000 reduced the area of leaves 1-6 (Figure 16). For example, L6 area was 22% higher in RTx7000 (67.4 cm2) than 6078-1 (55.3 cm2), although this difference was not significant at the P=0.05 level. The area of L6 ranged from 47.8 cm2 to 93.9 cm2 (LSD [0.05] = 21), with a heritability of 42.

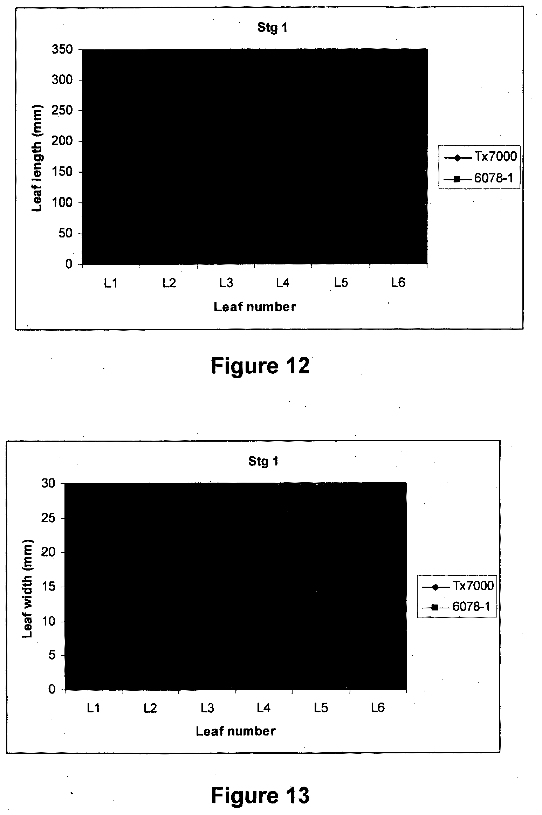

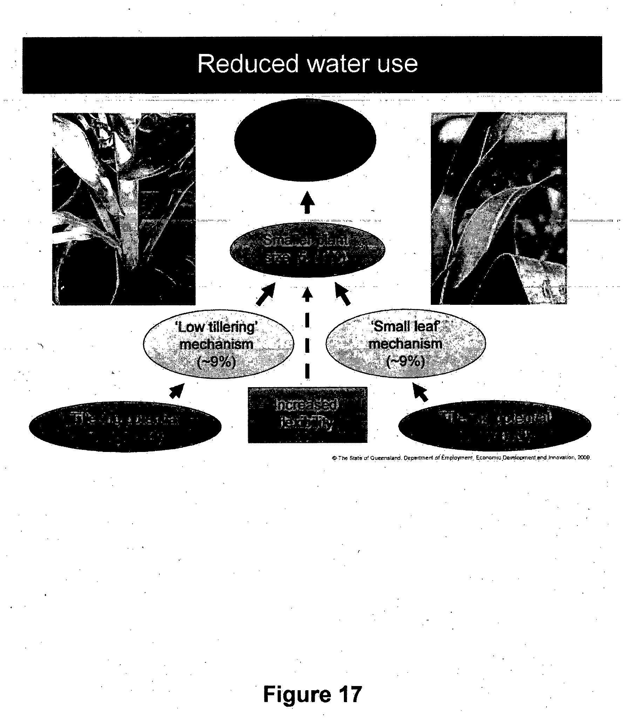

Differences in leaf area were due more to differences in leaf length (Figure 17) than leaf width (Figure 13). While leaf area increased exponentially with leaf number (Figure 16), leaf length increased linearly (Figure 12). The relationship between leaf width and number was parabolic (Figure 13). Hence the divergence in leaf area between 6078-1 and RTx7000 with increasing leaf number was due mainly to the divergence in leaf length between these genotypes. This suggests that the function of genes reducing leaf size is more likely associated with cell expansion (leaf length) than division (leaf width).

The allometric relationship in the Stgl fine-mapping population between the area of leaf (n) and the area of leaf (n+1) indicates a significant change at about Leaf 8 (concurrent with floral initiation). Thereafter, increases in leaf size occurred at a lesser rate.

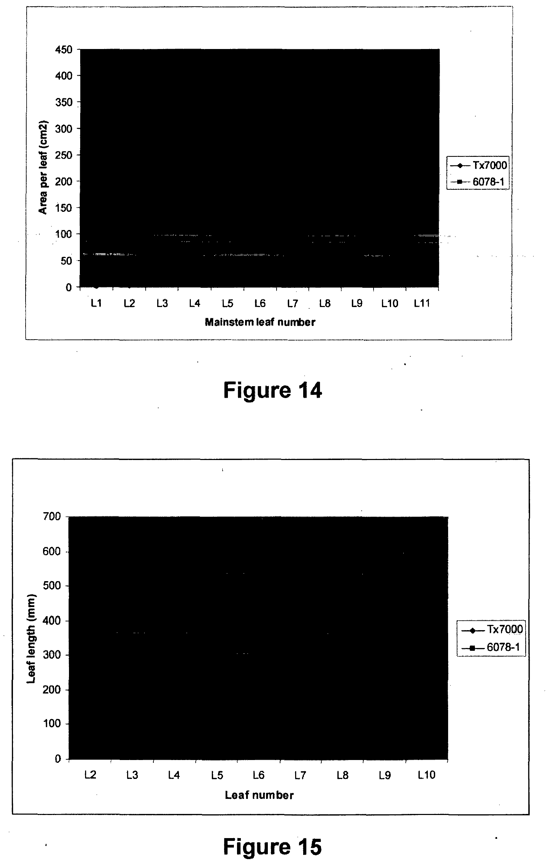

Introgressing the Stgl region into RTx7000 reduced the area of leaves 9-11 (Figure 14), as well as leaves 1-6 (discussed earlier). The area of L9 varied significantly (P=0.06) among genotypes, ranging from 234 to 300 cm2, with a heritability of 21 (Table 6). L9 area was 8% higher in RTx7000 (263 cm2) than 6078-1 (244 cm2) [Figure 14]. Similar trends were apparent for leaves 10 and 11.

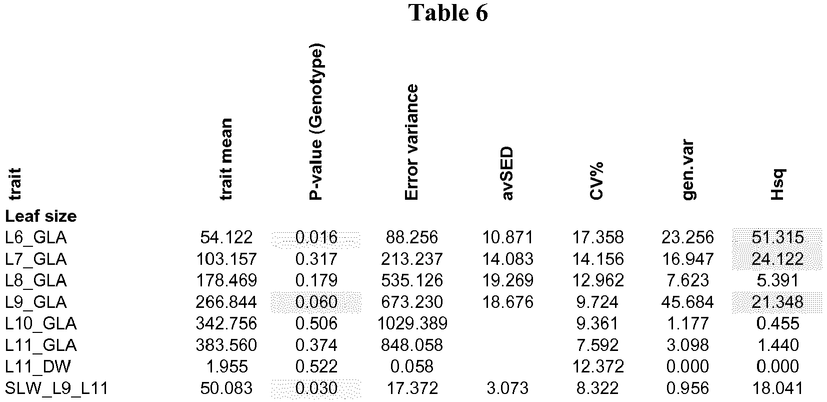

Table 6 shows a summary of predicted means, P-value and heritability of leaf size traits measured at the L11 harvest. Significant differences (P<0.05) are shaded in yellow while heritabilities >20 are shaded in green. GLA = green leaf area. DW = dry weight. SLW_L9_L11 = specific leaf weight.

Most of the variation in green leaf area at the Leaf 11 harvest was due to differences in tillering. However, leaves 9-11 were smaller in 6078-1 compared with RTx7000. These differences were significant (P<0.05). Note that 'low tillering' and 'small leaves' were both associated with the same region, indicating the possibility of a single gene controlling both canopy architecture traits.

### EXAMPLE 10

### Smaller leaves (fine-mapping studies in igloo)

Leaf number and length were linearly correlated for the parents of the Stgl fine-mapping population (Figure 15). Introgressing Stgl into RTx7000 resulted in a reduction in the length of leaves 8-10, with L10 being 7% shorter in 6078-1 than RTx7000 (550 vs. 592 mm).

For mapping purposes, the 'tails' of the Stgl fine-mapping population were selected. Two genotypes exhibited particularly long leaves (10604-1-157-5 and 10604-1-318-1) and three genotypes exhibited particularly short leaves (10604-1-222-1, 10604-1-501-327-3 and 6078-1).

Gain-of-function (short leaf) is achieved in recombinant 10604-1-222-1. The 'small leaf' gene is proposed to map to the same region as the 'low tillering' gene.

For mapping purposes, the 'tails' of the Stg1 fine-mapping population were selected (Figure 16). Three genotypes exhibited particularly long leaves (10604-1-157-5, 10604-1-318-1 and RTx7000) and two genotypes exhibited particularly short leaves (10604-1-222-1 and 6078-1).

Stepping up through the markers, gain-of-function (short leaf) is achieved in recombinant 10604-1-222-1. Hence, once again, the 'small leaf' gene maps to the same region as the 'low tillering' gene. Note that leaf length in L9 and L10 map to the same region.

An explanation is a single gene with multiple pleiotrophic effects. Enhanced availability of auxin would explain both the low tillering and small leaf size phenotypes observed in plants containing this region. An auxin efflux carrier component 5 gene is located in the target region and is therefore identified as a candidate.

### EXAMPLE 11

### Stay-green enhances canopy architecture plasticity via constitutive and adaptive responses

Increased water availability at anthesis is achieved via reduced water use due to two mechanisms (reduced tillering and smaller leaves) in plants containing the Stg1 region Figure 17). Both mechanisms, individually, appear to reduce canopy size by about 9%, on average. The 'low-tillering' mechanism dominates in low density environments when tillering potential is high. The 'small-leaf' mechanism dominates in high density environments when tillering potential is low. Combined, these two mechanisms provide crop plants with considerable plasticity to modify canopy architecture in response to the severity of water limitation.

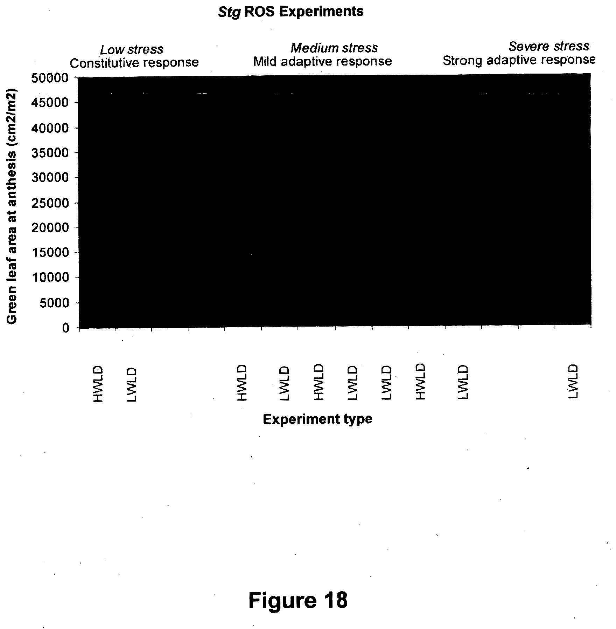

Stay- green exhibits both constitutive and adaptive responses (Figure 18). Canopy size in Stg1 is reduced by about 5%, even when water is not limiting (constitutive response). Canopy size is further reduced (adaptive response) in a mild drought (∼10%) and more severe drought (∼15%). Low tillering is primarily a constitutive response, although smaller leaf size in tillers in response to increasing water deficit is an adaptive response. Small leaf size is both a constitutive and adaptive response.

### EXAMPLE 12

### Link between reduced canopy size (via reduced tillering and smaller leaves) and reduced crop water use at anthesis.

Reduced crop water use at anthesis can be caused by a) a smaller canopy size with equivalent transpiration per unit leaf area, b) an equivalent canopy size with lower transpiration per unit leaf area, or c) a smaller canopy size and lower transpiration per unit leaf area. ROS and lysimetry studies indicate that under high water stress conditions, the Stgl region, and in particular the recombinant containing the Stgl candidate gene (10604-5), exhibited lower crop water use due to a smaller canopy size rather than lower transpiration per unit leaf area. High correlations (r2=0.9) between canopy size and crop water use were observed in ROS and lysimeter studies.

### (a) Water savings due to smaller leaf size

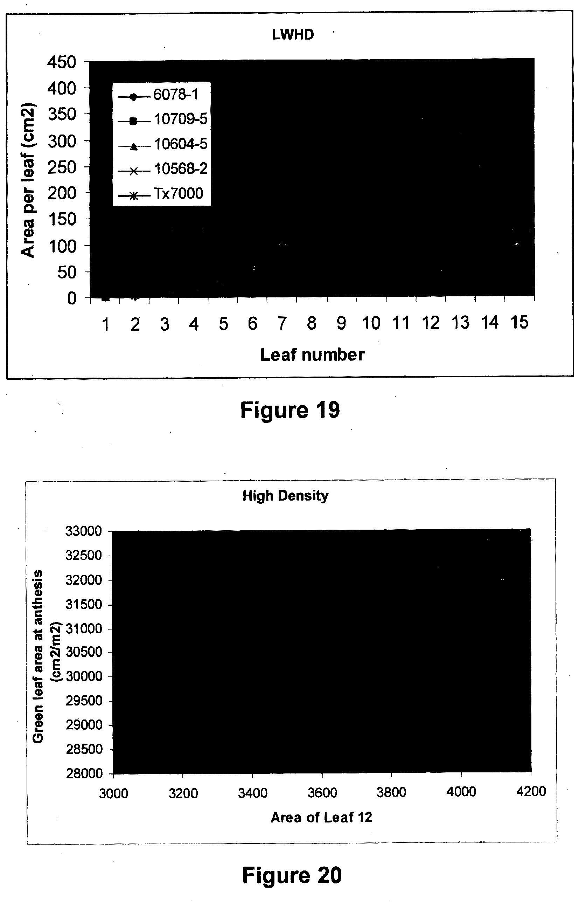

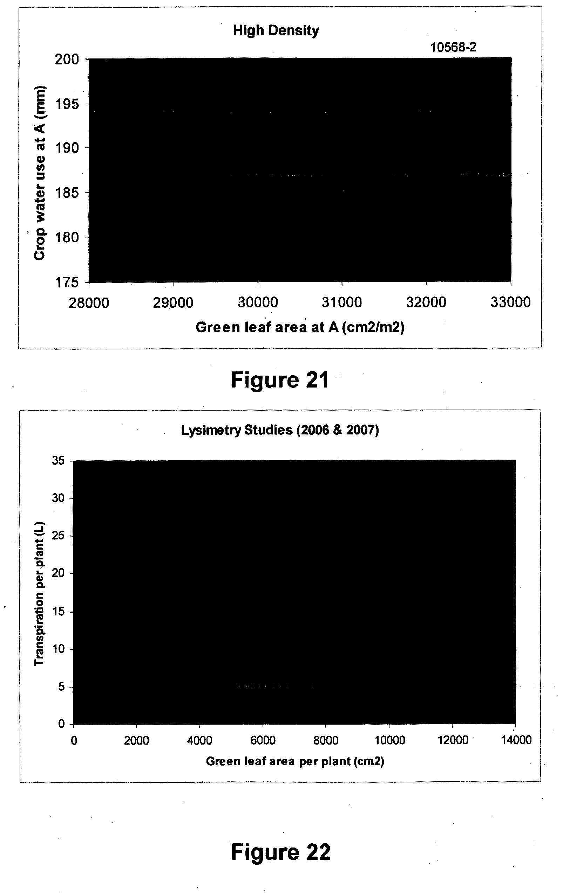

Tillering was negligible in this experiment due to the high crop density. Hence differences in canopy size were due to differences in leaf size (Figure 20), as evidenced by the high correlation between the size of Leaf 12 and the total green leaf area at anthesis (Figure 21).

In turn, green leaf area at anthesis was highly correlated with crop water use at anthesis (Figure 22). Relative to RTx7000, the two lines containing the Stgl candidate gene (6078-1 and 10604-5) both exhibited smaller leaves (L10-L13), lower green leaf area at anthesis (GLAA), and lower crop water use at anthesis.

### (b) Water savings due to reduced transpiration per unit leaf area (lysimetry studies)

Transpiration (T) is the product of leaf area (LA) and transpiration per leaf area (T/LA). Under high VPD conditions, LA was similar between Stgl and RTx7000 (11795 vs 11628 cm2), yet T/LA was less in Stgl than Tx7000 (2.60 vs 2.85), resulting in less water use per plant (T) in Stgl than RTx7000 (30.7 vs 32.81). Hence water savings in Stgl (in a high VPD environment) were achieved entirely by a reduction in T/LA, suggesting that this is a constitutive water conservation strategy conferred by Stg1. In this case, higher transpiration efficiency (TE) in Stgl was a consequence of equivalent biomass and lower transpiration.

A broader analysis comparing the four Stg QTL (Stg1, Stg2, Stg3 and Stg4) with RTx7000 helps to put the Stgl response in perspective. Under high VPD conditions, T/LA was positively correlated with T. However under low VPD conditions, T/LA was negatively correlated with T (r2=0.52). Green leaf area and transpiration were positively correlated under both low and high VPD conditions (Figure 22). In both experiments, the Stg QTLs reduced green leaf area and transpiration compared with RTx7000.

### (c) Water savings due to reduced leaf area

Transpiration (T) is the product of LA and T/LA. Under low VPD conditions, LA was 31% less in Stg1 than RTx7000 (4898 vs 7082 cm2). This was offset slightly by a 9% increase in T/LA in Stg1 compared with RTx7000 (5.15 vs 4.70). The net result was a 22% reduction in water use per plant (T) in Stgl compared with RTx7000 (25.6 vs 32.7 1), primarily due to reduced canopy size. The increase in T/LA exhibited by Stgl may itself be a drought adaptation mechanism, cooling the leaf and enabling photosynthesis to continue.

The plasticity in T/LA appears to be particularly important in the regulation of plant water status. Under high VPD conditions, reduced T/LA in Stgl was the key mechanism for reducing T and increasing TE. Under low VPD conditions, increased T/LA in Stg1 may have contributed to maintenance of leaf function via cooling.

Lysimetry studies on a Stgl fine-mapping subset provide additional insight into this region. Under high VPD conditions, LA per plant was less in 10604-5 (location of Stgl candidate gene) than RTx7000 (10283 vs 11628 cm2), yet T/LA was equivalent in 10604-5 and RTx7000 (∼2.86), resulting in less water use per plant in 10604-5 than RTx7000 (28.0 vs 32.8 1). Hence water savings in 10604-5 were achieved entirely by a reduction in canopy size.

Under low VPD conditions, LA per plant was less in all of the Stgl lines compared with RTx7000, resulting in water savings in all Stgl lines. Therefore it was difficult to fine-map this region, since all recombinants responded similarly to 6078-1.

### (d) Simulation of Agronomy

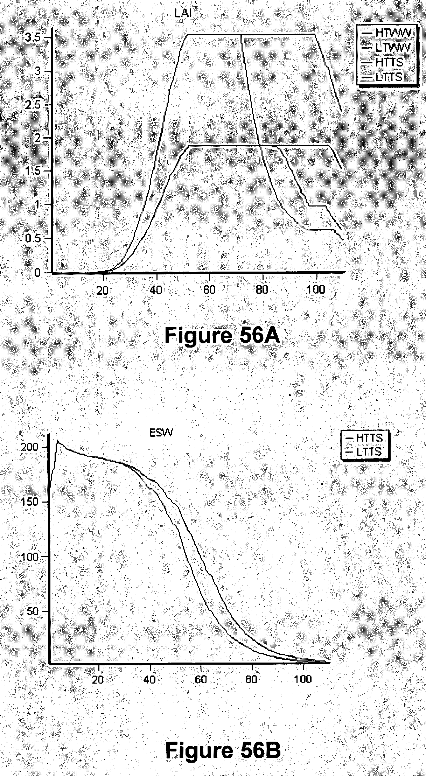

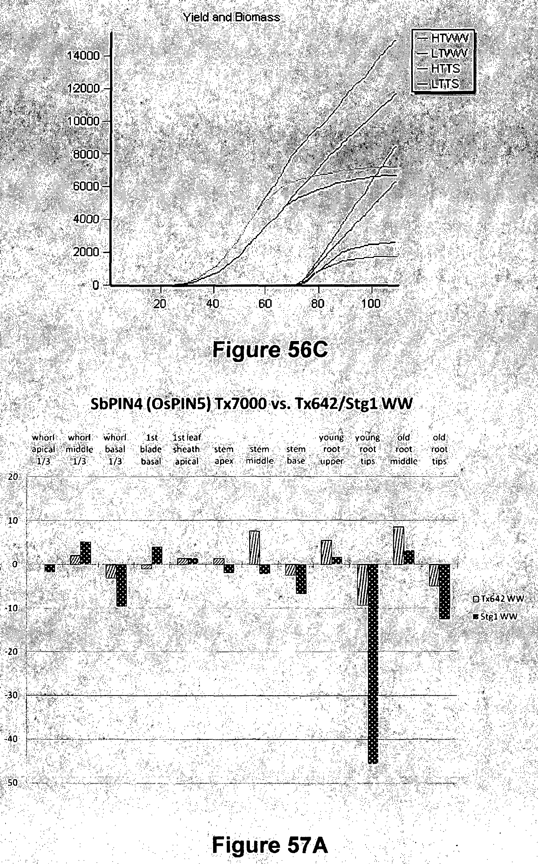

Buster planted at 5 plants/m2 with 1 m row spacing. Soil depth =1800mm; soil PAWC = 324mm; N non-limiting. Results are shown in Figures 56A through C.

### Treatments

- HT: High Tillering (2 tillers/plant)
- LT: Low Tillering (1 tiller/plant)
- WW: Well Watered (Start with 100% profile and rain fed)
- TS: Terminal Stress (Start with profile half full [162mm] and no rain after establishment).

### EXAMPLE 13

### Increased water availability at anthesis may also be achieved via increased water accessibility due to better water extraction and/or deeper or greater lateral spread of roots in plants containing the Stg1 region

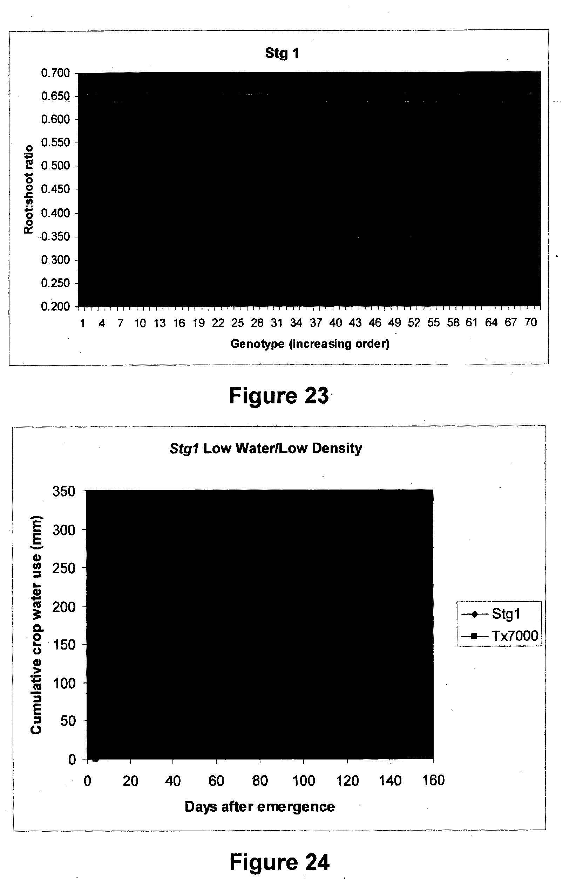

Root mass and root:shoot ratio (Figure 23) were higher in Stgl than RTx7000 at the Leaf 6 stage. There was considerable transgressive segregation for these traits in the Stgl fine-mapping population. The relation between root mass and root:shoot ratio highlights the opportunity for further genetic advance in these traits.

Root mass per leaf area ratio can be used as a drought adaptation index at the seedling stage since it integrates the capacity of the plant to access water (root mass) with the capacity of the plant to utilise water (leaf area). A higher index indicates a greater capacity to access water per unit leaf area. Stgl exhibited a higher root mass per leaf area ratio relative to RTx7000 due to both a higher root mass and a smaller leaf area.

The higher root mass per leaf area ratio exhibited by Stgl at the L6 stage may explain why it used more water early in crop growth (20-50 DAE) compared with RTx7000 under the LWLD treatment (Figure 24). It is not clear yet whether the increased water accessibility during grain filling exhibited by Stgl compared with RTx7000 (Figure 24) was due to better water extraction and/or greater root spread.

In a root chamber experiment at Gatton, Queensland, Australia (Van Oosterom et al., 2010 supra), the gravimetric lower limit of water extraction was 0.26% lower for A35 (stay-green) than AQL39 (senescent) hybrids. A35 contains the Stgl region whereas AQL39 does not. Assuming a bulk density of 1.3 g cm-3 and a soil depth of 150 cm, this could potentially increase available water in the field by >5 mm throughout the life cycle of the crop.

### EXAMPLE 14

### Stay-green enhances biomass per unit leaf area at anthesis. Assuming root mass is equivalent (or at least not significantly less), these differences could be explained by differences in transpiration per unit leaf area and/or transpiration efficiency

Mainstem biomass per unit leaf area (B/LA) at anthesis was ∼24% higher in Stgl than RTx7000 under low water stress (35.2 vs 26.2 g/m2/cm2) and high water stress (40.6 vs 31.4 g/m2/cm2) conditions (Table 7). Mainstem B/LA at anthesis was ∼14% higher under high water stress than low water stress conditions for both Stgl and RTx7000, i.e. B/LA increased with water deficit. Note that tiller B/LA was equivalent in Stgl and RTx7000 under low and high water stress conditions.

**Table 7**

| *Stg* status | No of lines | Mainstem biomass per unit leaf area at anthesis (g/m²/cm²)x1000 | | Tiller biomass per unit leaf area at anthesis (g/m²/cm²)x1000 | | Total biomass per unit leaf area at anthesis (g/m²/cm²)x1000 | |
|---|---|---|---|---|---|---|---|
| | | Low water stress | High water stress | Low water stress | High water stress | Low water stress | High water stress |
| *Stg1 region* | | | | | | | |
| 1 | 1 | 35.17 | 40.61 | 19.42 | 22.38 | 31.83 | 39.73 |
| 1,2 | 2 | 31.70 | 34.70 | 18.40 | 19.77 | 28.02 | 33.16 |
| 1,4 | 1 | 28.12 | 30.88 | 22.68 | 16.53 | 28.66 | 26.69 |

| *No regions* | | | | | | | |
|---|---|---|---|---|---|---|---|
| RTx7000 | 7 | 26.21 | 31.36 | 18.04 | 20.61 | 21.20 | 25.81 |
| | | | | | | | |
| LSD (0.05) | | 6.70 | 6.70 | 5.90 | 5.90 | 5.96 | 5.96 |

Table 7 shows mainstem, tiller and total biomass per leaf area for RTx7000 (recurrent parent) and a number of near-isogenic lines containing various Stgl introgressions grown under high and low water stress at Biloela, Queensland, Australia.

The detailed water use measurements suggest that the higher biomass per unit leaf area observed in Stgl lines at Biloela was probably be due to higher transpiration per unit leaf area rather than TE.

### Low water stress

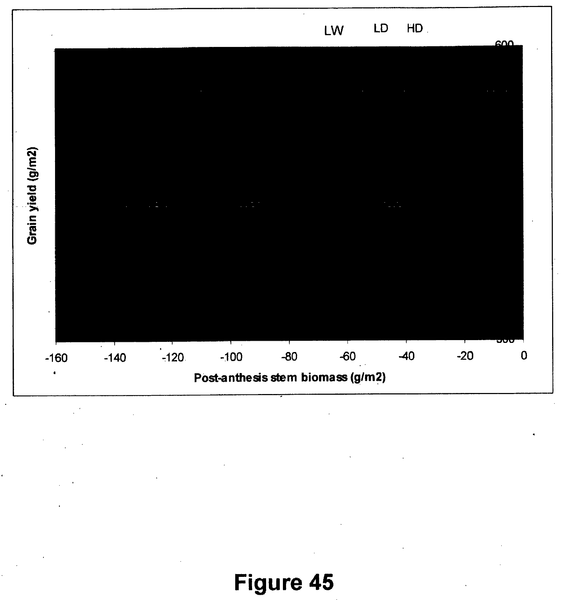

Stg1 and RTx7000 displayed equivalent B/LA under low water stress. However, T was ∼7% lower in Stg1, due to ∼10% lower T/LA which, in turn, increased TE by ∼9% (Figure 45). Therefore, Stgl maintained biomass but used less water compared with RTx7000.

### High water stress

Under high water stress, B/LA was ∼6% higher in Stg1 compared with RTx7000. B/LA was positively correlated with T/LA but not with TE. Hence, the higher B/LA displayed by Stg1 was due to higher T/LA. In general, B/LA was positively correlated with T/LA and negatively correlated with TE under high water stress.

In this case, Stgl used ∼22% less water than RTx7000 during the pre-anthesis period. Therefore, Stgl would have significantly more water available to fill grain, despite lower biomass at anthesis.

### EXAMPLE 15

### Increased TE via introgressing Stgl may be due to a) proportionally higher photosynthetic capacity compared with stomatal conductance, due to smaller, thinner and greener leaves, or b) a decrease in transpiration per leaf area while maintaining biomass per leaf area

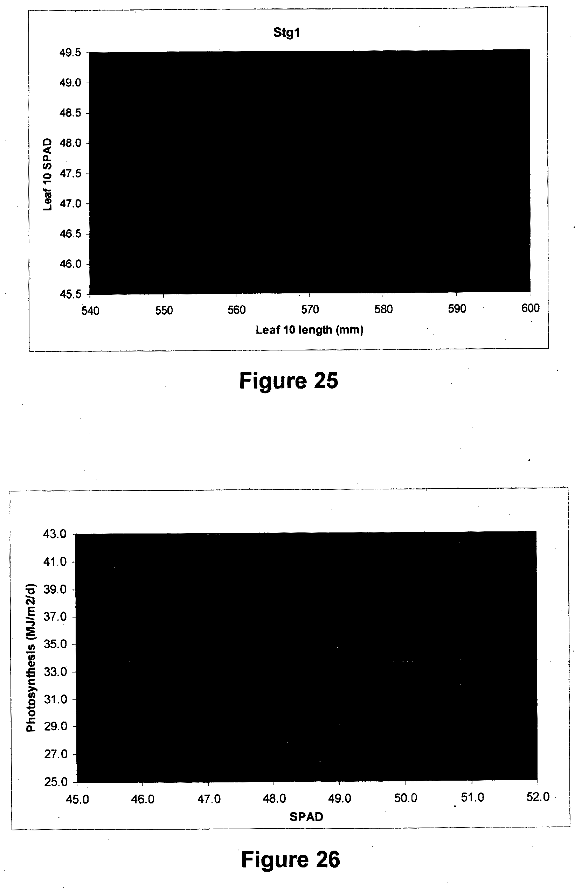

In the Stgl fine-mapping population, the length and greenness (SPAD) of Leaf 10 were highly negatively correlated (r2=0.72, Figure 25). Hence, decreasing the size of a leaf in this population increased the concentration of nitrogen in the leaf. Introgressing Stgl into a RTx7000 background decreased L10 length by ∼7% (from 592 to 550 mm) and increased L10 SPAD by ∼4% (from 47.1 to 48.9).

Greener leaves may increase photosynthetic capacity and therefore water use efficiency. In a subset of the Stgl fine-mapping population, photosynthesis increased with SPAD value until reaching a plateau at a SPAD of ∼48.5 (Figure 26). However, the line (6078-1) with the highest SPAD value (51.6) exhibited a relatively low rate of photosynthesis (32.1 MJ/m2/d). This result is either a) anomalous, or b) indicates a real decline in photosynthesis at high SPAD values.

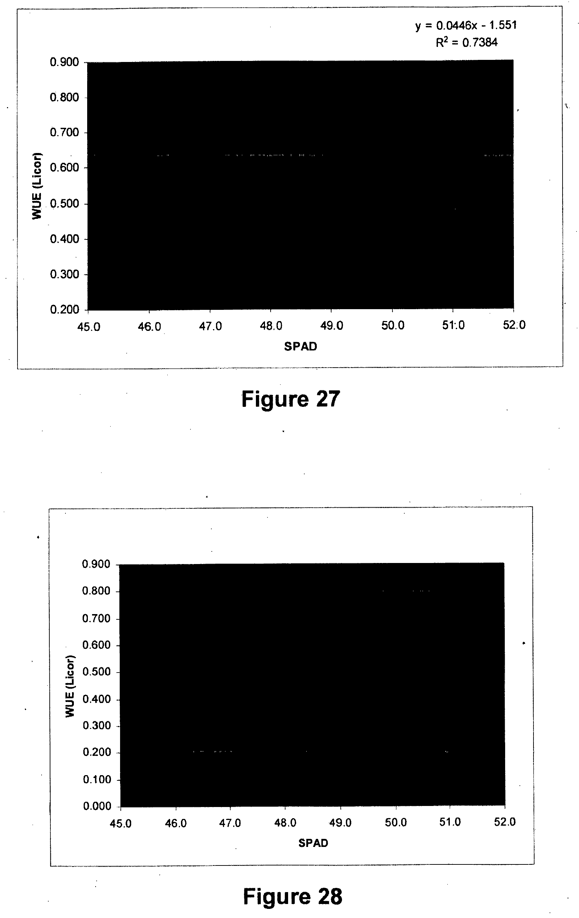

Leaf greenness (SPAD) and WUE (based on an index calculated for Licor software) were positively correlated in a subset of the Stgl fine-mapping population (Figure 27). None of the Stgl introgressions approached the value for 6078-1 (0.8), despite relatively high SPAD in the 10604-5 and 10709-5 isogenic lines (NILs).

Compared with RTx7000 and other Stg QTLs, Stgl exhibited a greener leaf (higher SPAD value) and higher WUE (based on an index calculated for Licor software) [Figure 28].

### EXAMPLE 16

### Increased TE via introgressing Stgl may be due to a) proportionally higher photosynthetic capacity compared with stomatal conductance, due to smaller, thinner and greener leaves, or b) a decrease in transpiration per leaf area while maintaining biomass per leaf area

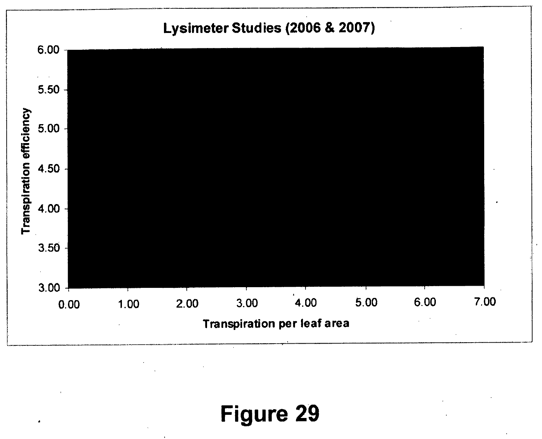

Transpiration efficiency (TE) was negatively correlated with transpiration per leaf area (T/LA) under low and high VPD conditions (Figure 29) in a set of Stg NILs, including the recurrent parent (RTx7000). However, the ranking of Stg NILs relative to RTx7000 interacted with VPD conditions. For example, T/LA in Stgl was lower relative to RTx7000 under high VPD conditions, yet higher than RTx7000 under low VPD conditions.

Under high VPD conditions, the slope of the negative correlation between T/LA and TE was steep, such that a slight decrease in T/LA from 2.9 mm/cm2 (Stg4) to 2.6 mm/cm2 (Stg1) resulted in a significant increase in TE from 4.2 g/m2/mm (Stg4) to 5.1 g/m2/mm (Stg1) [Figure 29]. The gradient was less steep under low VPD conditions such that a sixfold greater decrease in T/LA was required per unit increase in TE compared with high VPD conditions (1.2 vs 0.2 units). Note that for an equivalent TE, Stgl exhibited a higher T/LA than RTx7000 (5.1 vs 4.6 mm/cm2) under low VPD. This may provide a mechanism for Stgl leaves to remain cooler under certain environmental conditions.

### EXAMPLE 17

### Changes in transpiration per unit leaf area could be due to a) number of stomata, b) stomatal aperture, c) changes in the timing of stomatal opening and closing relative to VPD, and/or d) number of hair base cells (which affects the boundary layer and hence T/LA).

Introgressing Stgl into RTx7000 variously affected T/LA, depending on VPD conditions. Relative to RTx7000, Stgl increased T/LA by ∼9% under low VPD and decreased T/LA by ∼10% under high VPD. T/LA, *inter alia*, can be regulated by a) the number of stomata per unit leaf area, b) the size to the stomatal aperture, c) the timing of stomatal opening and closing, and/or d) the number of hair base cells (which affects the boundary layer and hence T/LA). Measurements of two of these four components (a and d) have been made. In one rainout shelter experiment, individual leaves were harvested from the high density treatment within the irrigated control, cuticles removed, and images taken of the cuticle surface. These images were used to determine a) the number of stomata per unit leaf area, b) the number of epidermal cells per unit leaf area, and c) the number of hair base cells per unit leaf area.

At the same time, transverse leaf sections were taken. Preliminary analysis of these data indicate that introgressing Stgl into RTx7000 modified leaf anatomy. Differences in the morphology of leaves (e.g. Leaves 7 and 10) are apparent between RTx7000 and Stg1. In this case, there were more and smaller bundle sheaths surrounding the vascular bundle in Stg1. The increased number of cells in the bundle sheath might also contribute to increased photosynthetic assimilation (PNAS 2007) and hence TE.

### EXAMPLE 18

### Increased water use during grain filling is achieved via (i) increased water availability at anthesis and (ii) increased water accessibility (better water extraction and deeper or greater lateral spread) during grain filling

### a) Increased water availability at anthesis

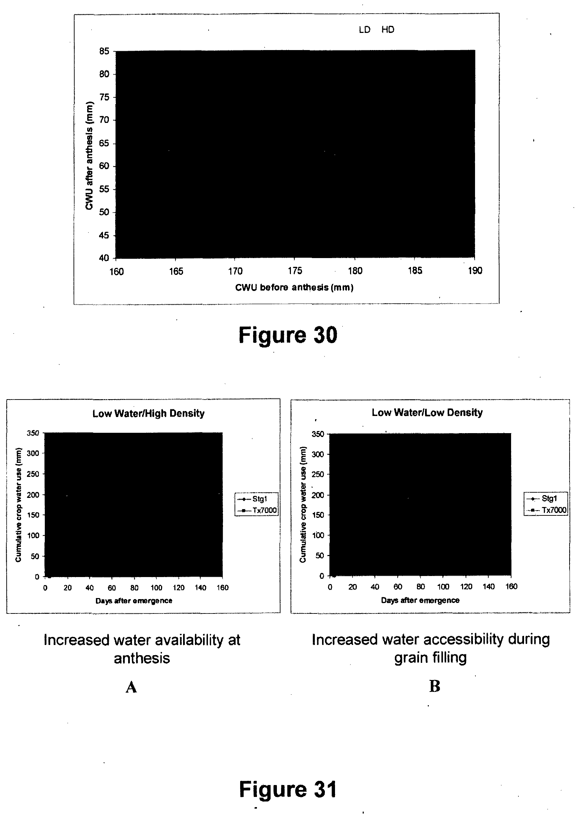

Crop water use (CWU) before anthesis was negatively correlated with CWU after anthesis in the ROS experiment (Figure 30). For example, saving 20 mm of water before anthesis (165 vs 185 mm) enabled the utilization of an additional 20 mm after anthesis (80 mm vs 60 mm). So all of the water conserved before anthesis was utilized by the crop after anthesis. Overall, a 25% increase in water use after anthesis in this experiment resulted in a 25% increase in grain yield (400 vs 300 g/m2). This translated to 50 kg/ha of grain for every additional mm of water available. While these data support the concept that increased water use during grain filling is achieved via increased water availability at anthesis, it does not explain about increased water accessibility during grain filling.

### b) Increased water accessibility during grain filling

Increased water use during the grain filling period was exhibited by Stgl under both low and high density treatments in the ROS experiment. This was due primarily to (i) increased water availability at anthesis under high density, and (ii) increased water accessibility during grain filling under low density (Figures 31A and B).

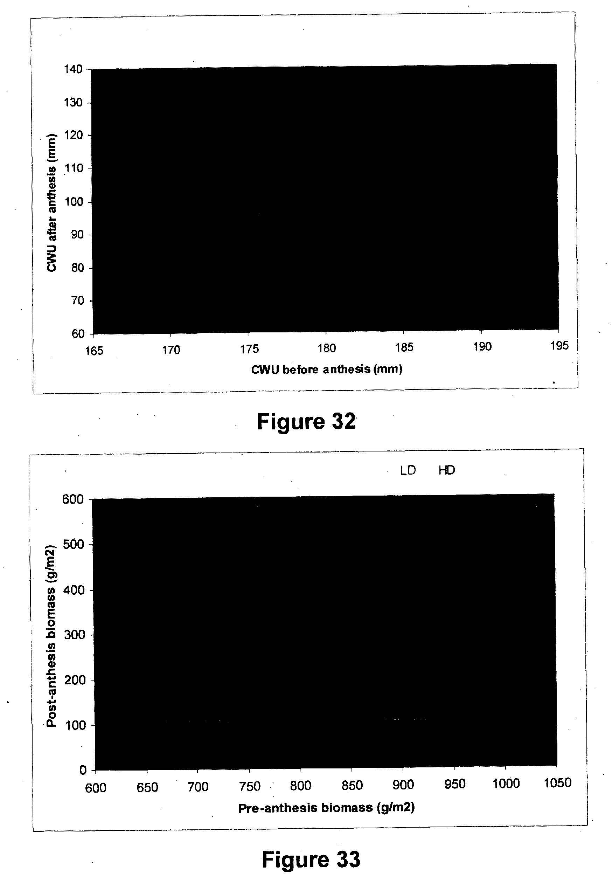

In a study of RTx7000 and four Stg NILs (Stg1, Stg2, Stg3 and Stg4), CWU before and after anthesis were negatively correlated in an ROS experiment under low density conditions (Figure 32). In this case, saving ∼25 mm of water before anthesis (168 vs 191 mm) contributed to the utilization of ∼50 mm after anthesis (135 mm vs 86 mm), indicating that both increased water availability (∼25 mm) and accessibility (∼25 mm) were equally important. However, Stgl was anomalous in this example, since high water use after anthesis was associated with high water use before anthesis. Explanations for this anomaly in Stgl are a) an error in the pre-anthesis water data, b) an error in the post-anthesis water data, or c) no errors in water measurement (Stg1 simply responded differently to the other NILs). An examination of the biomass data reveals that, for some reason, Stgl produced higher biomass before anthesis under LWLD compared with the other NILs, suggesting that the pre-anthesis water use patterns simply reflected biomass production in this experiment. Nonetheless, this example does provide evidence of increased water accessibility by Stg1 during the grain filling period.

### EXAMPLE 19

### Link between pre- and post-anthesis biomass production

Under low density (LD), reducing pre-anthesis biomass by 23% (from 700 to 640 g/m2) increased post-anthesis biomass more than twofold (from ∼200 to 425 g/m2). Under LD, Stgl produced similar pre-anthesis biomass to RTx7000 (∼610 g/m2), yet produced less post-anthesis biomass (265 vs 327 g/m2). However under HD, Stgl and RTx7000 produced similar pre-anthesis biomass (∼840 g/m2), yet Stgl produced more post-anthesis biomass (195 vs 17 g/m2).

The relation between GLAA and the pre:post anthesis biomass ratio is critical in the Stgl story. GLAA must be cut back to <3 to ensure the availability of adequate water for grain filling, and this is the critical role of the Stgl gene. In this experiment, Stgl reduced GLAA adequately to achieve a pre:post anthesis biomass ratio of <3 under LD, but not HD. Under HD, note that introgressing Stgl into RTx7000 reduced the GLAA from 31200 to 29300 cm2/m2, reducing the pre:post anthesis biomass ratio from 8.2 to 6.5 (but still not to <3). This highlights the importance of appropriate management strategies such as crop density in maximising limited water resources.

The negative relation between GLAA and post-anthesis stem mass is also critical to the Stgl story. Lower GLAA, and hence reduced water use at anthesis, was associated with higher post-anthesis stem mass (a component of lodging resistance). Introgressing Stgl into RTx7000 increased post-anthesis stem mass under both LD (marginal increase) and HD (significant increase) conditions.

The relation between the pre:post anthesis biomass ratio (PPBR) and post-anthesis biomass is instructive. The two density treatments provide a continuum in the range of PPBR from <2 to >8. Reducing PPBR from >8 to ∼3 resulted in a gradual increase in post-anthesis biomass. However, further reducing PPBR below 3 resulted in a relatively sharp increase in post-anthesis biomass, presumably because more water was available during grain filling when the PPBR ratio fell below 3. Introgressing Stgl into RTx7000 increased post-anthesis biomass under HD but not LD.

The relation between the pre:post anthesis biomass ratio (PPBR) and post-anthesis stem mass is equally instructive. Post-anthesis stem mass is a component of lodging resistance. Analysis of this component provides some understanding of how Stg introgressions affect lodging resistance. Reducing PPBR from >8 to ∼4 resulted in a gradual increase in post-anthesis stem mass. However, further reducing PPBR below 4 resulted in a relatively sharp increase in post-anthesis stem mass. Introgressing Stgl into RTx7000 increased post-anthesis stem mass under both LD (marginal increase) and HD (significant increase) conditions.

The relation between PPBR and grain yield was less clear in this experiment. While grain yield was higher in Stgl than RTx7000 under both densities, the higher yield could only be explained by lower PPBR in Stgl under HD.

Two Stgl introgressions were examined in this experiment: a) 6078-1 (the whole Stgl region), and b) 10946-5 (a recombinant covering about 1/3 of the Stgl region between markers Sb03QGM116 and Sb03QGM106).

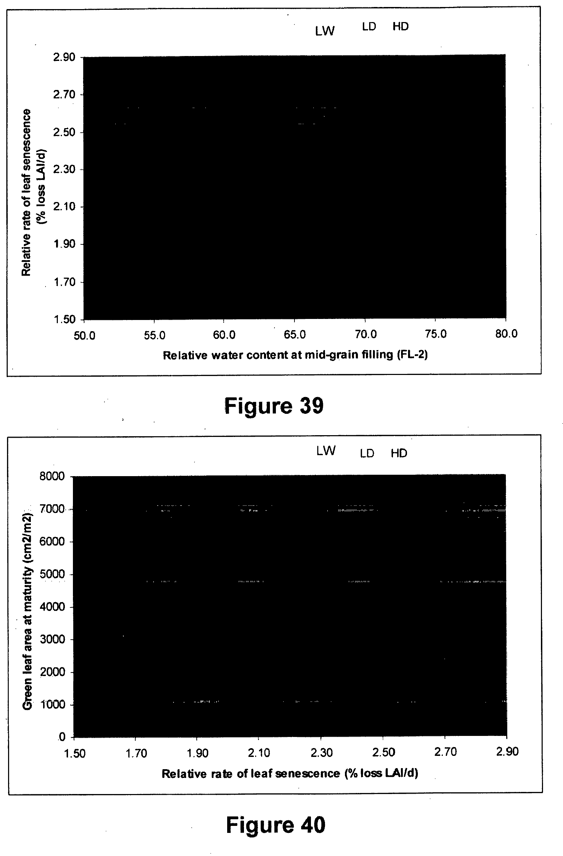

Reducing pre-anthesis biomass by 20% (from 920 to 735 g/m2) increased post-anthesis biomass by about 100% (from 200 to 400 g/m2) [Figure 40]. In general, pre-anthesis biomass in RTx7000=Stg2>Stg3=Stg4>Stg1 and post-anthesis biomass in Stg1>Stg3=Stg4>Stg2>RTx7000.

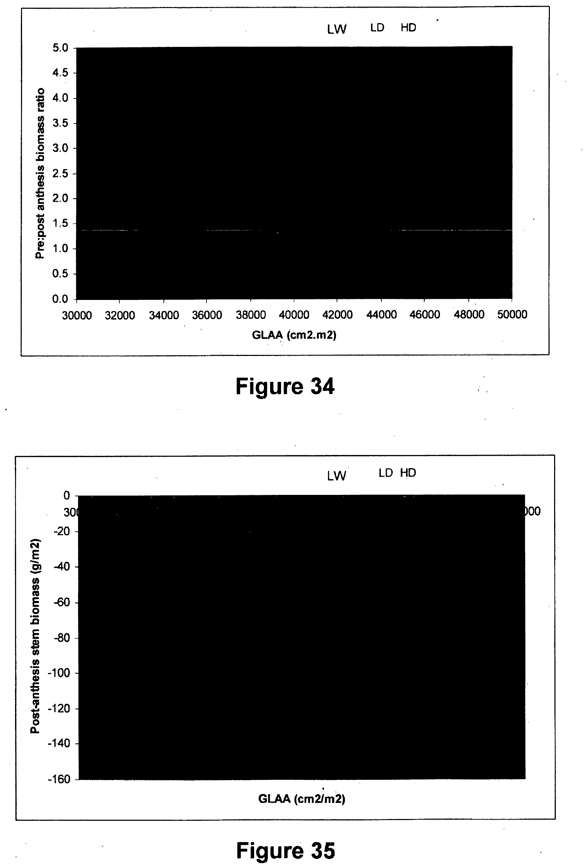

Canopy size, as evidenced by GLAA, largely determined the ratio of pre:post anthesis biomass (Figure 34). Under both high and low density treatments, introgressing Stgl into a RTx7000 background reduced GLAA which, in turn, reduced the ratio of pre:post anthesis biomass to <3, ensuring adequate water availability for grain filling under these water-limited conditions.

Canopy size, as evidenced by GLAA, was a determinant of post-anthesis stem mass (PASM) [Figure 35]. Under both high and low density treatments, introgressing Stgl or Stg1a into a RTx7000 background reduced GLAA which, in turn, increased PASM. Approximately 40 g/m2 more stem reserves were utilized under HD compared with LD, reflecting the higher stress imposed by this treatment.

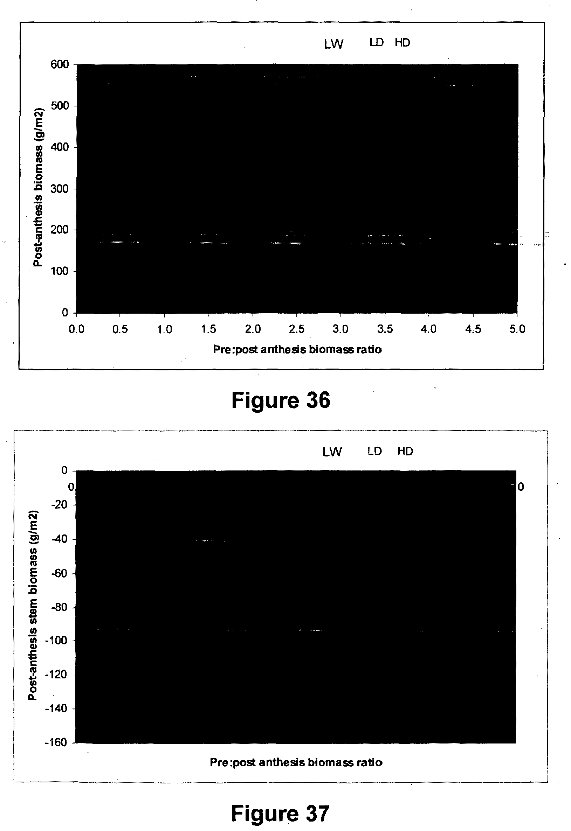

The relation between the pre:post anthesis biomass ratio (PPBR) and post-anthesis biomass (PAB) was strong (Figure 36). The two density treatments provide a continuum in the range of PPBR from <1.5 to >4. Reducing PPBR from ∼4.5 to ∼3.5 had no impact on PAB. However, further reducing PPBR below ∼3.5 resulted in a relatively sharp increase in PAB, presumably because more water was available during grain filling when the PPBR ratio fell below 3.5. Introgressing Stgl into RTx7000 decreased PPBR below 3 under HD and LD, thereby increasing PAB under both density treatments.

The pre:post anthesis biomass ratio (PPBR) also affected lodging resistance (Figure 37). In this case, post anthesis stem mass (PASM) is used as a surrogate for lodging resistance. Under high and low densities, PPBR was negatively correlated with post-anthesis stem biomass. That is, a high pre:post anthesis biomass ratio increased the amount of stem reserves remobilized during grain filling, thus reducing stem biomass and increasing the likelihood of lodging. Compared with RTx7000, Stg1a significantly reduced the amount of stem reserves mobilized under LD (∼5 vs 65 g/m2) and HD (∼80 vs 140 g/m2). Compared with RTx7000, Stgl significantly reduced the amount of stem reserves mobilized under HD (∼80 vs 140 g/m2), but not LD. The extent of stem reserves mobilized was greater under HD than LD, reflecting the greater water deficit under HD. For example, the difference in stem reserve mobilization between HD and LD was twofold in RTx7000 (about 140 vs 70 g/m2).

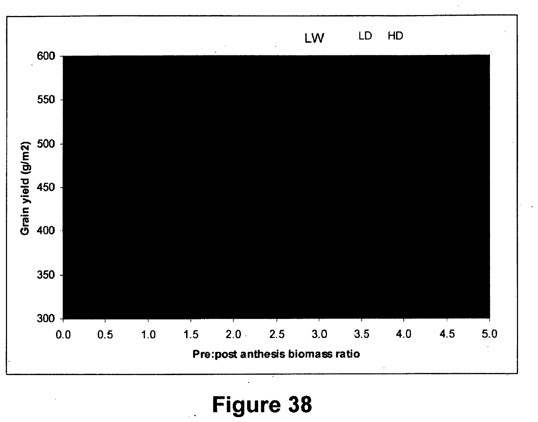

Grain yield remained low (at a benchmark of ∼4.2 t/ha) until the pre:post anthesis biomass ratio fell below ∼3 (HD) or ∼2.5 (LD) [Figure 38]. Below these critical values, grain yield increased significantly for each incremental reduction in these ratios, with the rate of increase in grain yield being slightly higher under LD than HD. This suggests that post-anthesis water availability was closely linked to pre-anthesis GLAA and biomass, and that a certain reduction in GLAA was required to ensure adequate water availability for grain filling. Under both densities, Stgl reduced the pre:post anthesis biomass ratio below the critical levels, resulting in yield increases of 28% (LD) and 22% (HD), relative to RTx7000. These data provide a critical link between Stgl gene action (reduced canopy size at anthesis) and grain yield under terminal drought. Note that the Stg1a introgression had some impact on reducing PPBR (HD and LD) and increasing grain yield (LD only) relative to RTx7000, but not to the same extent as Stg1. Hence, there is not strong evidence from this experiment that the key Stg1 gene resides within the Stg1a region. This supports evidence presented earlier, since the strongest candidate for Stgl (SbPIN4) is located above the Stg1a introgression.

Four Stgl introgressions were examined in this experiment: a) 6078-1 (the whole Stgl region); b) 10709-5 (a recombinant covering about 1/3 of the Stgl region); c) 10604-5 (a recombinant covering about 3/4 of the Stgl region); and d) 10568-2 (a recombinant covering almost 1/2 of the Stgl region).

Under low density (LD), pre-anthesis biomass varied by only ∼5% (from 522 to 552 g/m2) among genotypes, yet post-anthesis biomass varied almost twofold (from 173 to 313 g/m2). This suggests that the considerable differences in post-anthesis biomass were affected by something other than pre-anthesis biomass, e.g. differences in water accessibility. For example, 10709-5 and RTx7000 both produced ∼550 g/m2 of pre-anthesis biomass, yet the Stgl recombinant (10709-5) produced ∼60% more post-anthesis biomass.

Under high density (HD), pre- and post-anthesis biomass were highly negatively correlated. Introgressing Stgl into RTx7000 reduced pre-anthesis biomass by 9% and increased post-anthesis biomass by 23%.

Crop water use (CWU) at anthesis better discriminated between genotypes than did pre-anthesis biomass. Combining the HD and LD data, post-anthesis biomass (PAB) remained low (at a benchmark of ∼150 g/m2) until the CWU at anthesis fell below ∼180 mm. Below this critical value, PAB increased for each incremental reduction in CWU down to a level of 175 mm, with PAB plateauing at about 310 g/m2. Further reductions in CWU at anthesis below 175 mm did not result in additional PAB.

Canopy size, as evidenced by GLAA, largely determined the ratio of pre:post anthesis biomass (PPBR). Under both high and low density treatments, introgressing Stgl (or particular recombinants such as 10709-5) into a RTx7000 background reduced GLAA which, in turn, reduced the ratio of pre:post anthesis biomass, thereby increasing water availability for grain filling under these water-limited conditions. The PPBR value for 6078-1 appears anomalous (too high) since this genotype is placed well above the GLAA/PPBR regression line.

Canopy size, as evidenced by GLAA, was a determinant of post-anthesis stem mass (PASM). Under the high density treatment, introgressing Stg1 (or Stg1 recombinants such as 10604-5 and 10709-5) into a RTx7000 background reduced GLAA which, in turn, increased PASM. Approximately, 60 g/m2 more stem reserves were utilized under HD compared with LD, reflecting the higher stress imposed by this treatment.

The relation between the pre:post anthesis biomass ratio (PPBR) and post-anthesis biomass (PAB) was strong. The two density treatments provide a continuum in the range of PPBR from <2 to >5, although the slope of the regression was greater for LD than HD. Under HD, reducing PPBR from ∼6 to ∼3.5 resulted in a gradual increase in PAB from ∼130 g/m2 (RTx7000) to ∼180 g/m2 (10604-5). Further reducing PPBR below ∼3 under LD resulted in a steeper increase in PAB, presumably because more water was available during grain filling when the PPBR ratio fell below 3.

The pre:post anthesis biomass ratio (PPBR) also affected lodging resistance. In this case, post anthesis stem mass (PASM) is used as a surrogate for lodging resistance. Under high and low densities, PPBR was negatively correlated with post-anthesis stem biomass. That is, a high pre:post anthesis biomass ratio increased the amount of stem reserves remobilized during grain filling, thus reducing stem biomass and increasing the likelihood of lodging. Compared with RTx7000, Stg1 significantly reduced the amount of stem reserves mobilized under HD (∼100 vs 160 g/m2). The extent of stem reserves mobilized was greater under HD than LD, reflecting the greater water deficit under HD. For example, the difference in stem reserve mobilization between HD and LD was more than twofold in RTx7000 (about 160 vs 60 g/m2). Interestingly, PASM increased with decreasing PPBR over the whole range of PPBR (1.5 - 6), whereas grain yield, and to a lesser extent PAB, only increased when PPBR fell below ∼3. This suggests that relatively small water savings before anthesis were still able to improve lodging resistance, although greater water savings were required before grain yield responded.

Grain yield remained low (at a benchmark of ∼3.1 t/ha) until the pre:post anthesis biomass ratio fell below ∼3. Below this critical value, grain yield increased significantly for each incremental reduction in this ratio. Since none of the Stgl introgressions reduced the PPBR to <3 under HD, no yield benefits were realised from Stgl in this treatment. Under LD, some of the Stgl introgressions reduced PPBR below the critical level, resulting in yield increases of 12% (10568-2) and 5% (10709-5), relative to RTx7000. These data provide a critical link between Stgl gene action (reduced canopy size at anthesis) and grain yield under terminal drought.

CWU during grain filling remained low (at a benchmark of ∼60 mm) until the pre:post anthesis biomass ratio fell below ∼3.5 (Figure 39). Below this critical value, CWU during grain filling increased significantly for each incremental reduction in this ratio. Since none of the Stg1 introgressions reduced the PPBR to <3.5 under HD, no increases in CWU during grain filling were realised from Stgl recombinants in this treatment. Under LD, some of the Stg1 introgressions (and RTx7000) reduced PPBR below the critical level, increasing CWU during grain filling up to ∼80 mm compared with the HD baseline (∼60 mm).

The relation between CWU during grain filling and grain yield was positive (Figure 40). However, the correlation between these parameters was relatively low (r2=0.23), probably due to the highly variable nature of soil water measurements in the field.

### EXAMPLE 20

### Delayed leaf senescence (stay-green) is a consequence of higher plant water status during grain filling (due to increased water use)

Plant water status was determined on FL-2 (two leaves below the flag) at mid-grain filling using two methodologies: leaf water potential (LWP) and relative water content (RWC). LWP was measured in the field with a pressure bomb. Following determination of LWP in the field, a sample of the same leaf was placed on ice and, within a few minutes, taken to a laboratory some 300 m away for determination of RWC by standard methods.

The RWC of FL-2 was negatively correlated with the relative rate of leaf senescence at mid-grain filling under both high and low densities in a set of Stg NILs including the recurrent parent. Correlations for HD and LD were parallel, but offset by about 0.35 units of leaf senescence, i.e. for a given level of RWC, say 70, the relative rates of leaf senescence were 2.1 and 2.45 for LD and HD, respectively. Introgressing the Stgl region into RTx7000 increased RWC at mid-grain filling (FL-2) and decreased the relative rate of leaf senescence under both HD and LD, although the impact was greater under HD.

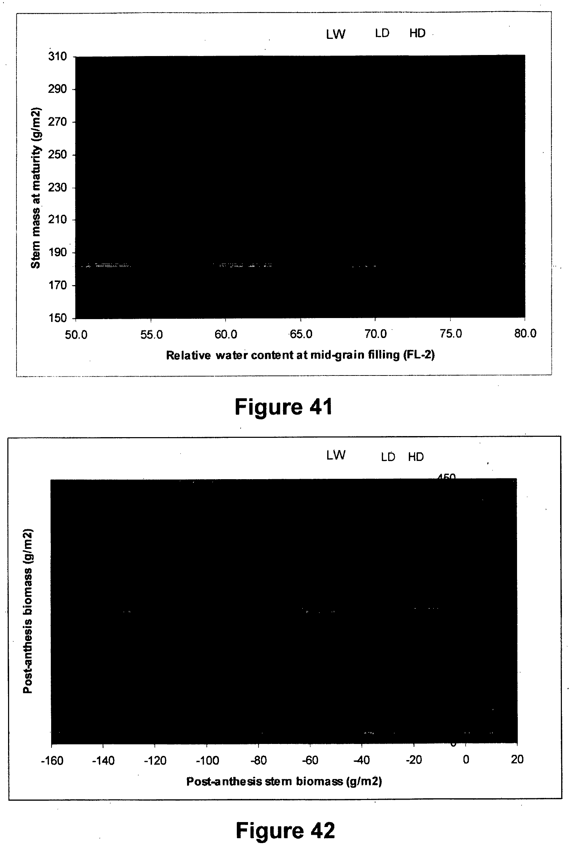

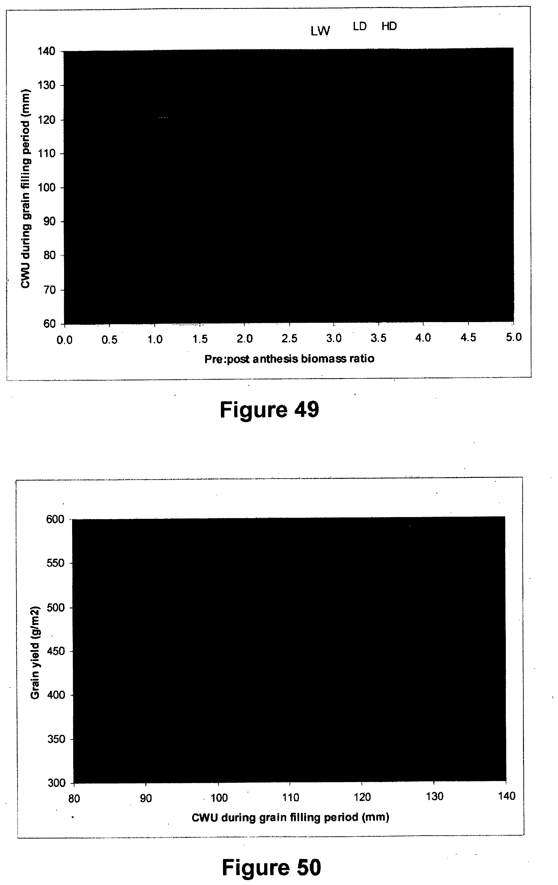

In turn, the relative rate of leaf senescence was highly negatively correlated with green leaf area at maturity (GLAM) under HD and LD (Figure 49), with higher rates of leaf senescence exhibited under HD. Stgl produced more than twice as much GLAM as RTx7000 under HD (2106 vs 859 cm2/m2) and 19% more GLAM under LD (2145 vs. 1725 cm2/m2), see also Figure 42.

### EXAMPLE 21

### Increased lodging resistance is a consequence of higher plant water status during grain filling (due to increased water use)

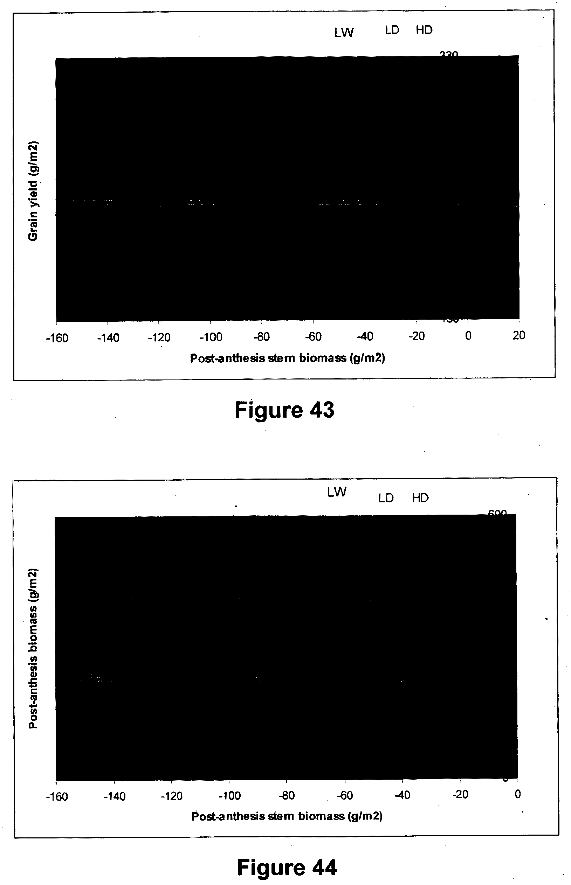

Higher stem mass at maturity is a component of lodging resistance. RWC at mid-grain filling (FL-2) was highly negatively correlated with stem mass at maturity in a set of Stg NILs grown under water-limited conditions at two crop densities (Figure 43). Introgressing Stgl into RTx7000 increased RWC at mid-grain filling (FL-2) and stem mass at maturity under HD and LD, with a greater impact under HD. Relative to RTx7000, Stg1 increased stem mass at maturity by 9% under LD (224 vs. 203 g/m2) and 29% under HD (286 vs. 204 g/m2). Hence the benefit of Stg1, in terms of lodging resistance, increased with the level of water deficit.

Post-anthesis biomass is mainly comprised of a) post-anthesis stem mass (PASM), a measure of stem reserve mobilization and a component of lodging resistance, and b) grain yield. Grain-growers require that both grain yield and lodging resistance be maximized, i.e. they do not want one at the expense of the other. Post-anthesis stem mass was highly linearly correlated with PAB under HD and LD conditions (Figure 44). While Stgl had little impact on PASM under the milder drought (LD), the amount of dry mass translocated from the stem during grain filling was much less in Stgl compared with RTx7000 (85 vs. 139 g/m2) under the more severe drought (HD). This resulted in greater stem mass at maturity in Stgl relative to RTx7000 (286 vs. 204 g/m2) which, in turn, should have increased lodging resistance.

While the correlations between PASM and PAB were high under HD and LD (Figure 43), the correlations between grain yield and PAB were low under both densities (Figure 44). Introgressing Stgl into RTx7000 increased grain yield under HD and LD, although PAB was only higher under HD. Note that the higher grain yield in Stgl compared with RTx7000 (290 vs. 184 g/m2) under HD was achieved with significantly less stem reserves in Stg1 than RTx7000 (85 vs. 139 g/m2), indicating that carbon demand during grain filling was largely met by current photo-assimilation and stem reserves in Stg1 and RTx7000, respectively.

The potential trade-off between PASM and grain yield is highlighted in Figure 45. Under HD, Stg1 achieved the highest grain yield of any introgression (290 g/m2) while keeping stem losses to 85 g/m2. Relative to RTx7000, Stg1 optimized the trade-off between PASM and grain yield. Further reductions in stem reserve utilization by Stg24 (38 g/m2) resulted in a lower grain yield (263 g/m2). Under LD, Stg1 also achieved a high grain yield relative to RTx7000 (282 vs. 214 g/m2) while utilising slightly less stem reserves (46 vs. 50 g/m2). Therefore under both densities, Stg1 attained higher grain yield and lodging resistance than RTx7000.

The relation between PASM and stem mass at maturity was relatively flat for the various Stg introgressions under HD and LD, although RTx7000 fell below the regression line in both cases. For a given level of stem reserve utilization (e.g. ∼140 g/m2 under HD or 50 g/m2 under LD), introgressing a Stg region into RTx7000 significantly increased stem mass at maturity, suggesting that some other factor (e.g. stem strength) in addition to the amount of stem reserves utilized was important.

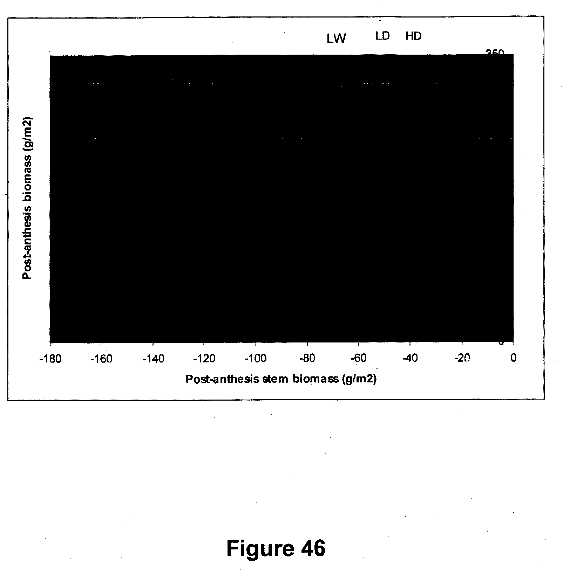

Post-anthesis stem mass (PASM) was highly linearly correlated with PAB under HD and LD conditions (Figure 46). The HD and LD correlations were almost parallel, although offset by about 50 g/m2, i.e. for a given level of PAB, say 300 g/m2, PASM was ∼50 g/m2 less in HD than LD (-100 vs. -50 g/m2). This reflects the higher level of stress in the HD treatment. Under HD, both Stg1 and Stg1a utilized ∼80 g/m2 of stem reserves compared with almost 140 g/m2 in RTx7000, yet Stg1 produced ∼28% more PAB than Stg1a for equivalent stem reserve utilisation. Under LD, Stg1a and to a lesser extent Stg1, both increased PAB relative to RTx7000. However, while Stg1a used ∼60 g/m2 less stem reserves than RTx7000, Stg1 used ∼20 g/m2 more stem reserves than RTx7000.

Grain yield was positively correlated with PAB under HD and LD. Under HD, Stg1 outyielded RTx7000 by 24% although Stg1a was equivalent to RTx7000 in grain yield. Under LD, Stg1 and Stg1a outyielded RTx7000 by 42% and 20%, respectively.

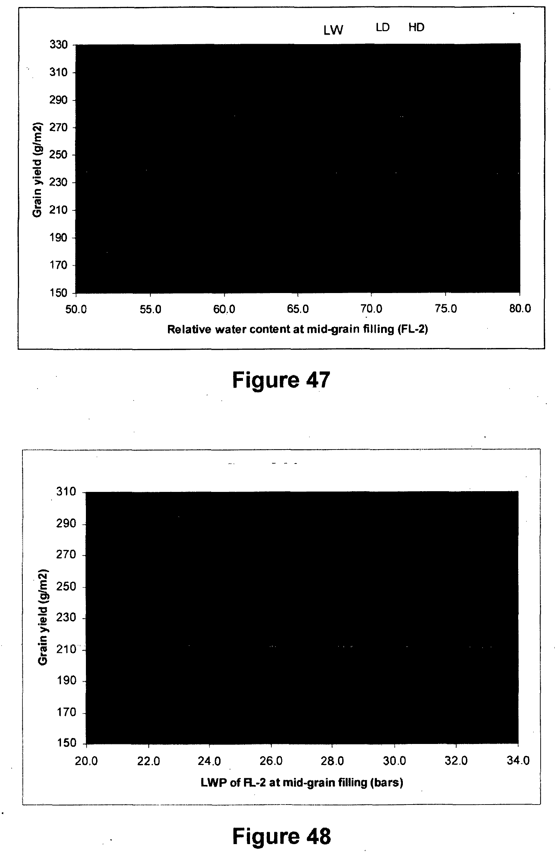

In this experiment, there was no trade-off between PASM and grain yield, since the correlation between these parameters was positive and linear for both crop densities (Figure 47). Under HD, Stg1 exhibited the highest stem mass and grain yield of all the Stg introgressions, highlighting that grain yield and lodging resistance are not mutually exclusive.

The relation between PASM and stem mass at maturity was positively correlated under HD and negatively correlated under LD. Under HD, PASM and stem mass at maturity were both significantly higher in Stg1 than RTx7000. Under LD, stem mass at maturity was higher in Stg1 than RTx7000 (314 vs. 271 g/m2), although Stg1 utilized more stem reserves compared with RTx7000 (87 vs 66 g/m2). Overall, Stg1 increased stem mass at maturity by 22% (HD) and 16% (LD) relative to RTx7000. Also, Stg1a utilized significantly less stem reserves than RTx7000 under both crop densities.

Post-anthesis stem mass (PASM) was highly linearly correlated with PAB under HD and LD conditions (Figure 48). Genetic variation in utilization of post-anthesis stem reserves ranged from ∼30-110 g/m2 under LD, and from ∼100-160 g/m2 under HD, reflecting the higher level of stress in the HD treatment. Under HD, the Stg1 parent (6078-1) and two of the Stg1 recombinants (10604-5 and 10709-5) utilized significantly less stem reserves compared with RTx7000 (∼100 vs.160 g/m2), yet produced more PAB than RTx7000 (∼170 vs. 130 g/m2). Under LD, only one of the Stg1 recombinants (10568-2) utilized significantly less stem reserves than RTx7000 (∼30 vs. 60 g/m2), and produced more PAB than RTx7000 (∼310 vs. 230 g/m2).

Grain yield was positively correlated with PAB under LD and negatively correlated under HD, although overall (combining HD and LD), the relationship was positive, with RTx7000 (HD) as an outlier.

In this experiment, there was no trade-off between PASM and grain yield, since the correlation between these parameters was positive and linear for both crop densities. Under HD, the Stg1 parent (6078-1) exhibited high stem mass and grain yield compared with the other Stg1 introgressions. RTx7000 was an anomaly under HD, exhibiting low stem mass and high grain yield. Under LD, 10709-5 and 10568-2 exhibited high stem mass and grain yield relative to RTx7000.

The relation between PASM and stem mass at maturity was positively correlated under both densities. Under HD, PASM and stem mass at maturity were both significantly higher in 6078-1 and 10709-5 than RTx7000. Under LD, only one Stg1 recombinant (10568-2) exceeded RTx7000 in PASM and stem mass at maturity.

### EXAMPLE 22

### Higher grain yield is a consequence of higher plant water status during grain filling

Introgressing Stg1 into a RTx7000 background increased plant water status at mid-grain filling, as indicated by a) higher relative water content (RWC) in FL-2 under LD and HD lower leaf water potential (LWP) in FL-2 under LD and HD. Overall, plants were more stressed under LD than HD in this experiment, evidenced by lower RWC under LD. However, the beneficial impact of Stg1 on plant water status was more dramatic under HD. where RWC was 26% higher in Stg1 than RTx7000.

RWC at mid-grain filling in FL-2 was positively correlated with grain yield under HD and LD. At higher levels of plant water stress (RWC<73), grain yield was higher under LD than HD for a given level of RWC. RWC and grain yield were higher in Stg1 than RTx7000 under both crop densities. For example in Stg1 under HD, a 26% increase in RWC was associated with a 58% increase in grain yield, relative to RTx7000.

The leaf water potential (LWP) of FL-2 at mid-grain filling was negatively correlated with grain yield under HD and LD conditions (Figure 49). Under both densities, Stg1 exhibited a lower LWP (less stressed) and higher grain yield relative to RTx7000. These data, together with the RWC data, provide strong evidence for a link between higher plant water status during grain filling and enhanced grain yield due to the Stg1 region.

### EXAMPLE 23

### Higher grain yield and larger grain size are consequences of increased water availability during grain filling

Critical to the hypothesis on Stg1 function is the link between pre- and post-anthesis water use and, subsequently, the link between post-anthesis water use and grain yield. The Stg1 gene is of little value at the field level is there is no link between increased water availability during grain filling and either grain yield or grain size.

First, it is important to establish the link between the pre:post anthesis biomass ratio (PPBR) and crop water use (CWU) during grain filling. CWU during grain filling remained low (at a benchmark of ∼85 and 95 mm for LD and HD, respectively) until the pre:post anthesis biomass ratio fell below ∼3 and 2.5 for LD and HD, respectively (Figure 50). Below this critical value, CWU during grain filling increased significantly for each incremental reduction in this ratio. Under both densities, Stg1 introgressions reduced the PPBR sufficiently to significantly increase CWU relative to RTx7000.

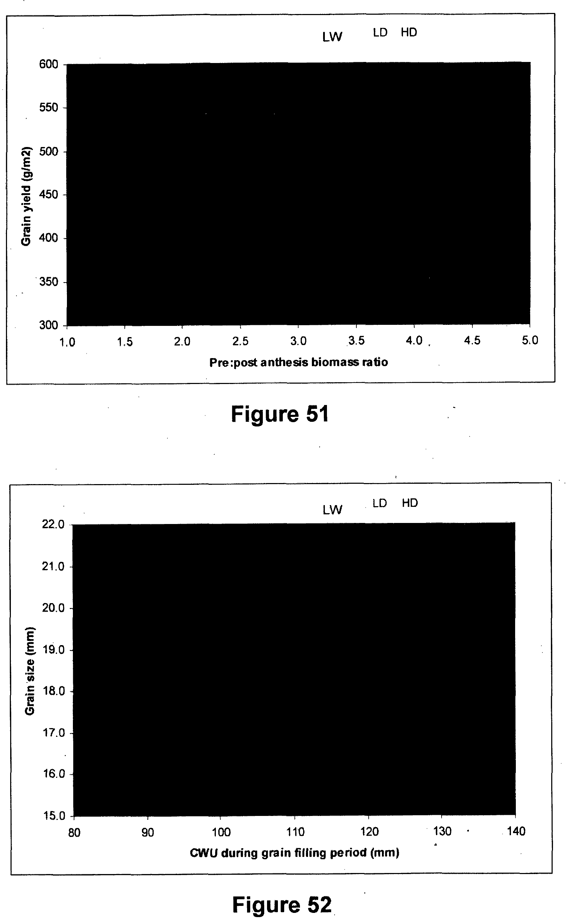

Second, it is important to show the link between CWU during grain filling and grain yield. In general, these parameters were positively associated in a ROS experiment, apart from two distinct outliers under LD (Stg2 and Stg3) [Figure 51]. Introgressing Stg1 into RTx7000 increased both CWU during grain filling and grain yield under both crop densities.

Finally, the link between PPBR and grain yield under water-limited conditions completes the picture (Figure 51). Grain yield remained low (at a benchmark of ∼410 g/m2) until the pre:post anthesis biomass ratio fell below ∼2.7 (Figure 52). Below this critical value, grain yield increased significantly for each incremental reduction in this ratio. Under both densities, Stg1 introgressions reduced the PPBR sufficiently to significantly increase grain yield relative to RTx7000. These data provide a critical link between Stg1 gene action (reduced canopy size at anthesis) and grain yield under terminal drought.

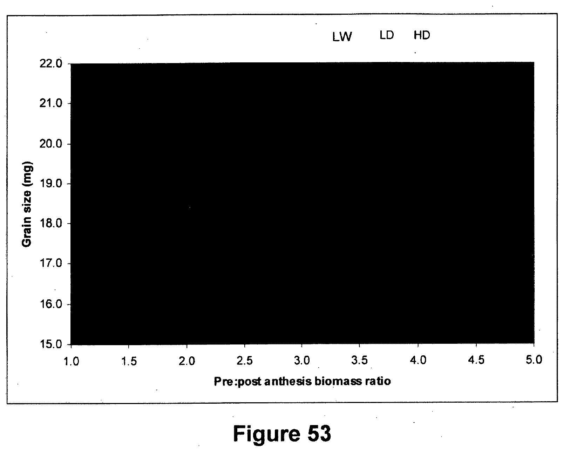

CWU during grain filling was positively correlated with grain size under both HD and LD treatments (Figure 53). Introgressing Stg1 into RTx7000 significantly increased grain size under HD, but not LD.

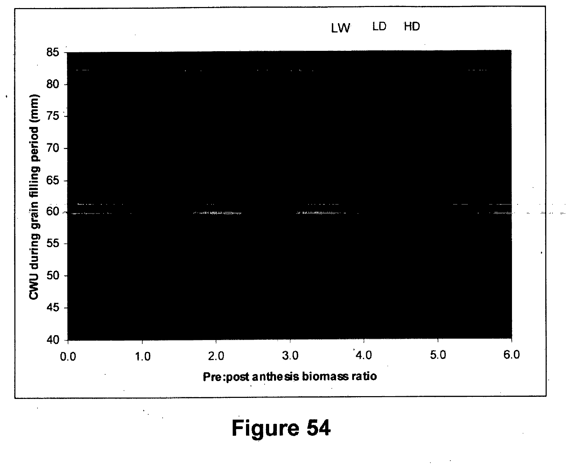

The relation between PPBR and grain size under water-limited conditions highlights the importance of water conservation before anthesis as a determinant of grain size (Figure 54). Grain size remained low (at a benchmark of ∼17 and 17.5 mg for HD and LD, respectively) until the pre:post anthesis biomass ratio fell below ∼2.7 (Figure 54). Below this critical value, grain size increased significantly for each incremental reduction in this ratio. Under HD but not LD, Stg1 introgressions reduced the PPBR sufficiently to significantly increase grain size relative to RTx7000. These data provide a critical link between Stg1 gene action (reduced canopy size at anthesis) and grain size under terminal drought.

CWU during grain filling remained low (at a benchmark of ∼58 mm for HD) until the pre:post anthesis biomass ratio fell below ∼3.5. Note that the PPBR did not drop below the critical threshold in any genotype under HD, hence CWU during grain filling remained relatively low for all genotypes in this treatment. However, as genotypes fell below this critical value in the LD treatment, CWU during grain filling increased significantly for each incremental reduction in this ratio. Only one Stg1 recombinant (10709-5) increased CWU during grain filling relative to RTx7000.

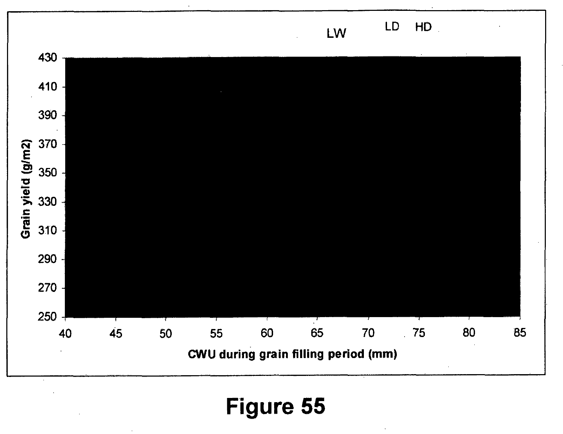

In general, CWU during grain filling and grain yield were positively correlated using a combined data set from the HD and LD treatments, with genotypes using more water and producing more grain under LD. However, the relation between these parameters was not very strong. Nor did particular Stg1 recombinants consistently out-yield RTx7000 in HD and LD treatments. See Figure 55.

### EXAMPLE 24

### QTL and PIN gene analysis

### QTL analysis

Stay-green QTL data were collected from 7 studies (Crasta et al., 1999; Hausmann et al., 2002; Kebede et al., Theoretical and Applied Genetics 103:266-276, 2001; Srininvas et al., Theor Appl Genet 118:703-717, 2009; Subudhi et al., Theor App Genet 101:733-741, 2000; Tao et al., Theor Appl Genet 100:1225-1232, 2000; Xu et al., Genome 43:461-469, 2000). From the 7 studies, 47 individual QTL were identified and projected onto the sorghum consensus map (Mace et al., BMC Plant Biol. 9:13, 2009). Where estimated Confidence Intervals (CI) of QTL for the same trait overlapped, those QTL were grouped into a meta QTL. Nine meta-QTL for stay-green were identified in this way. QTL for the same trait were classified as separate QTL if their CI had no region in common and mean QTL location were less than or equal to 15cM away from each other.

Statistical Machine Learning (SML) QTL analysis (Bedo et al., BMC Genetics 9:35, 2008) was conducted on a set of over 500 entries on the DEEDI sorghum pyt males trial. 23 QTL identified with a probability <0.05 were also plotted onto the consensus map.

### PIN gene analysis

All available PIN genes in rice and Arabidopsis were searched for via NCBI (www.ncbi.org). In total, sequence for 9 rice PIN genes (OsPIN1, OsPIN1b, OsPIN1c, OsPIN2, OsPIN3a, OsPIN3b, OsPIN4, OsPIN5, OsPIN6) and 3 Arabidopsis PIN genes (AtPIN1, AtPIN2, AtPIN4) were identified. All genes (protein sequence) were BLASTed against the sorghum WGS (www.gramene.org) and the top 100 hits were identified. The score (S value: a measure of the similarity of the query to the sequence shown), E-value (the probability due to chance, that there is another alignment with a similarity greater than the given S score), %ID and length of sequence homology for each of the 1200 hits were collated. The relationship between the 4 measures was analyzed and the S score was selected as the main measure to assess likelihood of sequence similarity. Following an analysis of the distribution of S score values, 3 S score categories were identified (>1000; >499 and <1000; <499) and a list of 11 sorghum genes with scores >499 (i.e. in the first 2 categories) was produced (Table 8).

**Table 8**

| **Sorghum Gene ID** | **Target gene** | **JGI Annotation** |
|---|---|---|
| Sb02g029210 | OsPIN6 | "similar to Os09g0505400 protein" |
| Sb03g029320 | OsPIN3a | "similar to Probable auxin efflux carrier component 3a" |
| Sb03g032850 | OsPIN1 | "similar to Putative uncharacterized protein" |
| Sb03g037350 | OsPIN5 | "similar to Probable auxin efflux carrier component 5" |
| Sb03g043960 | OsPIN6 | "similar to Probable auxin efflux carrier component 6" |
| Sb04g028170 | OsPIN1 | "similar to Auxin efflux carrier component 1" |
| Sb05g002150 | OsPIN1b | "similar to Probable auxin efflux carrier component 1b" |
| Sb07g026370 | OsPIN4 | "similar to Probable auxin efflux carrier component 4" |
| Sb10g004430 | OsPIN1 | "similar to Putative auxin efflux carrier component 3b" |
| Sb10g008290 | OsPIN1c | "similar to Probable auxin efflux carrier component 1c" |
| Sb10g026300 | OsPIN2 | "similar to Probable auxin efflux carrier component 2" |

### Comparisons

Of the 11 PIN orthologues identified, 10 (90.9%) aligned with known QTL for stay-green. Only one of the 11 sorghum PIN genes (Sb03g043960 on SBI-03, did not align to any reported QTL. Of the 79 QTL plotted (23 SML-QTL and 56 from the literature/meta-QTL), 30 (38%) aligned with the PIN orthologs.

### EXAMPLE 25

### Analysis of PIN2, PIN3, PIN4 and PIN5

The stay-green source BTx642 (B35), and near-isogenic lines (NILs) containing the Stg1, Stg2, Stg4 QTLs, as well as the contrasting senescent line Tx7000 were grown in root pipes in a glasshouse.

The aim of the experiment was to measure expression levels of genes that are identified herein as stay-green gene candidates under well-watered conditions and after a drought stress has been imposed on the plants to see whether there were any differences in expression in the stay-green compared with the senescent plants.

The experiment was divided into two parts: an early drought stress (Exp1) and a late drought stress (Exp2).

Results from the early drought stress experiment are shown in Table 9.

**Table 9**

| Gene | GeneID | Rice Homologue | Stg QTL | Comment |
|---|---|---|---|---|
| SbPIN2 | Sb03g029320 | OsPIN3a | Stg2 | Stg2 Candidate |
| SbPIN3 | Sb03g032850 | OsPIN1 | Stg1 | Upper Stg1 QTL |
| SbPIN4 | Sb03g037350 | OsPIN5 | Stg1 | Stg1 Candidate |
| SbPIN5 | Sb03g043960 | OsPIN6 | Stg1 | Lower Stg1 QTL |

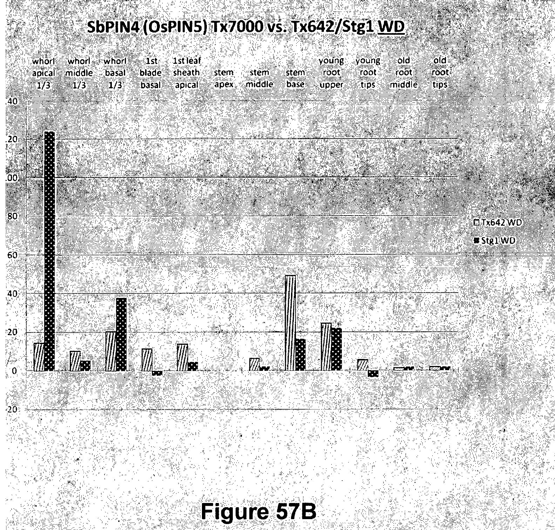

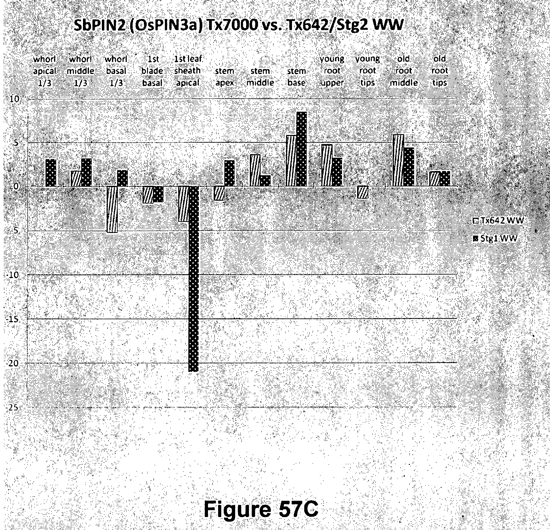

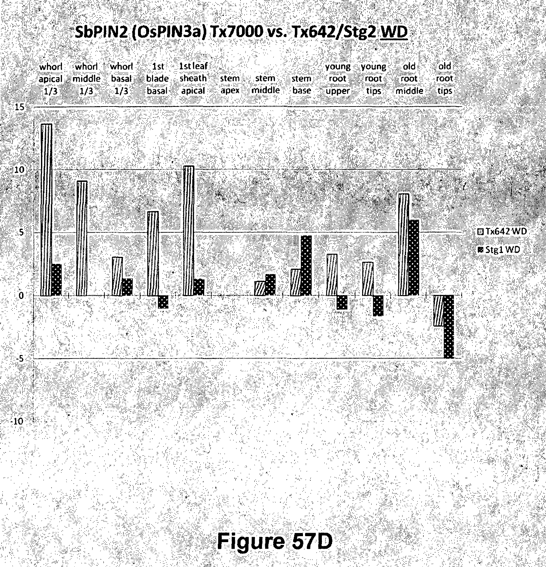

The main differences in expression of these PIN genes are summarised below (Table 10 and in Figures 57A through D).

**Table 10**

| **Gene** | **Main Tissues** | **Response to WD** | **Differential Expression** |
|---|---|---|---|
| SbPIN4 (Stg 1 candidate) | WW: low in leaves, high in roots | Lower expression in most tissues | WW: Higher in Tx7000 vs BTx642 and Stg1 in roots (Fig.1) |
| | WD: low in leaves, high in roots | | WD: Higher in Stg1 and BTx642 vs Tx7000 in leaves, stems and upper roots (Fig. 2) |
| SbPIN2 (Stg2 candidate) | WW: high in leaves, low in roots | Increase in roots, decrease in stems | WW: Higher in Stg2/BTx642 vs Tx7000 in stem and roots (Fig. 3) |
| | WD: high in leaves, low in roots | | WD: higher in BTx642 and to lesser extent Stg2 vs Tx7000 in most tissues except old roots (Fig. 4) |
| SbPIN3 | Limited variation among tissues | Limited response to WD | WD: slightly higher in BTx642/Stg1 vs Tx7000 |
| SbPIN5 | WW: high in leaves, low in roots | Increase in roots, not much change in leaves | WW: Higher in Stg1 (and to much lesser extent Tx642) vs Tx7000 in upper young roots especially |
| | WD: high in leaves, low in roots | | WD: Higher in Stg1 (and to lesser extent) BTx642 vs Tx7000 in stem |

Emerging patterns were identified for SbPIN4 and SbPIN2, Stg1 and Stg2, respectively.

In both cases the expression of these genes was higher in stay-green lines compared to the senescent line in response to water deficit.

These two PIN genes showed differences in tissue specificity. SbPIN4 was generally (across all conditions) more highly expressed in roots and stems and less expressed in leaves, while SbPIN2 generally showed higher expression in leaves and stems and lower expression in roots).

### BIBLIOGRAPHY

Andrade et al., Crop Sci. 42:1173-1179, 2002
Bedo et al., BMC Genetics 9:35, 2008
Benkova et al., Cell 115:591-602, 2003
Borrell et al., Crop Sci. 40:1026-1037, 2000a
Borrell et al., Crop Sci. 40:1037-1048, 2000b
Borrell and Hammer, Crop Sci. 40:1295-1307, 2000
Carraro et al., Plant Physiology 142:254-264, 2006
Christopher et al., Aust. J. Agric. Res. 59:354-364, 2008
Crasta et al., Molecular and General Genetics 262:579-588,1999
Forestan and Varotto, Plant Physiology, 2009
Friml et al., Current Opinion in Plant Biology 6:7-12, 2003
Garud et al., Int. Sorghum and Millets Newsl. 43:63-65, 2002
Hammer et al., Aust. J. Agric. Res. 48:649-655, 1997
Hammer, Agric. Sci. 19:16-22, 2006
Harris et al., J. Exp. Bot. 58:327-338, 2007
Hausmann et al., Theoretical and Applied Genetics 106:133-142, 2002
Henderson et al., Aust. J. Plant Physiol. 25:111-123, 1998
Henzell et al., Australia Int. Sorghum and Millets Newsl. 38:1-9, 1997
Jiao et al., Nature Genetics 42:541-544, 2010
Jordan et al., Theor. Appl. Genet. 106:559-567, 2003
Kashiwagi et al., Plant Physiology and Biochemistry 44:152-157,2006
Kassahun et al., Euphytica 72:351-362, 2010
Kebede et al., Theoretical and Applied Genetics 103:266-276, 2001
Leyser, Current Biology 16:R4242-R433, 2001
Mace et al., BMC Plant Biol. 9:13, 2009
Manschadi et al., Funct. Plant. Biol. 33:823-837, 2006
Miura et al., Nature Genetics 42:545-549, 2010
Mortlock and Hammer, J. Crop Prod. 2:265-286, 1999
Papanov et al., Trends in Plant Science 10(4):170-177, 2005
Passioura, J. Aust. Inst. Agric. Sci. 43:117-120, 1977
Rashotte et al., Plant Cell 13:1683-1697, 2000
Reddy et al., Euphytica 159:191-198, 2008
Reinhardt et al., Plant Cell 12:507-518, 2000
Rosenow et al., Agric. Water Manag. 7:207-222, 1983
Sadras and Connor, Field Crops Res. 26:227-239, 1991
Spano et al., J. Exp. Bot. 54:1415-1420, 2003
Springer, Nature Genetics 42:475-476, 2010
Srininvas et al., Theor Appl Genet 118:703-717, 2009
Subudhi et al., Theor Appl Genet 101:733-741, 2000
Tao et al., Theor Appl Genet 100:1225-1232, 2000
Tenkouano et al., Theor. Appl. Genet. 85:644-648, 1993
Turner, J. Exp. Bot. 55:2413-2425, 2004
Van Oosterom et al., Field Crops Res. 115:19-28, 2010a
Van Oosterom et al., Field Crops Res. 115:29-38, 2010b
Van Oosterom and Hammer, Field Crops Res. 108:259-268, 2008
Wang et al., Molecular Plant 2(4):823-831, 2009
Xin et al., Field Crops Res. 111:74-80, 2009
Xu et al., Genome 43:461-469, 2000
Zheng et al., Plant Breed 128:54-62, 2009

## Claims

1. A method for generating a genetically modified plant which uses water more efficiently than a non-genetically modified plant of the same species, the method comprising introducing by genetic engineering using recombinant means genetic material which encodes an SbPIN4 protein to confer a stay-green phenotype, which phenotype includes a shift in water use to the post-anthesis period or increased accessibility of water during crop growth or increased transpiration efficiency resulting in increased harvest index and grain yield under water-limited conditions, wherein said SbPIN4 protein is encoded by Sorghum Gene ID Sb03g037350 from bp 65310051 to bp 65313194.

2. The method of claim 1, further comprising introducing by genetic engineering using recombinant means genetic material which encodes SbPIN2.

3. The method of claim 2, wherein said SbPIN2 protein is encoded by Sorghum Gene ID Sb03g029320 from bp 57449784 to 57453744.

4. The method of any one of claims 1 to 3 wherein the genetically modified plant is a sorghum plant, or is selected from wheat, oats, maize, barley, rye and rice, or is selected from abaca, alfalfa, almond, apple, asparagus, banana, bean-phaseolus, blackberry, broad bean, canola, cashew, cassava, chick pea, citrus, coconut, coffee, corn, cotton, fig, flax, grapes, groundnut, hemp, kenaf, lavender, mano, olive, onion, pea, peanut, pear, pearl millet, potato, ramie, rapeseed, ryegrass, soybean, strawberry, sugar beet, sugarcane, sunflower, sweet potato, taro, tea, tobacco, tomato, triticale and yam.

5. The method of any one of claims 1 to 4 wherein the stay-green phenotype further includes a phenotype selected from enhanced canopy architecture plasticity, reduced canopy size, enhanced biomass per unit leaf area at anthesis, higher transpiration efficiency, increased water use during grain filling, reduced pre- and post-anthesis biomass production and delayed senescence, or further includes larger grain size.

6. Use of a genetic material which encodes a SbPIN4 protein for generating by recombinant means a genetically modified plant which uses water more efficiently than a non-genetically modified plant of the same species, wherein the said genetically modified plant has a stay-green phenotype which includes a shift in water use to the post-anthesis period or increased accessibility of water during crop growth or increased transpiration efficiency resulting in increased harvest index and grain yield under water-limited conditions, and wherein said SbPIN4 protein is encoded by Sorghum Gene ID Sb03g037350 from bp 65310051 to bp 65313194.

## Patentansprüche

1. Verfahren zum Erzeugen einer genetisch veränderten Pflanze, die Wasser effizienter nutzt als eine nicht genetisch veränderte Pflanze der gleichen Art, wobei das Verfahren ein Einführen eines genetischen Materials, das ein SbPIN4-Protein kodiert, durch Gentechnik, die rekombinante Mittel verwendet, umfasst, um einen grün-bleiben Phänotyp zu übertragen, wobei der Phänotyp eine Verschiebung des Wasserverbrauchs zu einer Nach-Anthese-Periode oder eine erhöhte Zugänglichkeit von Wasser während des Pflanzenwachstums oder eine erhöhte Transpirationseffizienz, die in einem erhöhten Ente-Index und Kornertrag unter eingeschränkten Wasserbedingungen resultiert, einschließt, wobei das SbPIN4-Protein durch Sorghum Gen ID Sb03g037350 von bp 65310051 bis bp 65313194 kodiert ist.

2. Verfahren nach Anspruch 1, weiter umfassend ein Einführen eines genetischen Materials, das SbPIN2 kodiert, durch Gentechnik, die rekombinante Mittel verwendet.

3. Verfahren nach Anspruch 2, wobei das SbPIN2-Protein durch Sorghum Gen ID Sb03g029320 von bp 57449784 bis 57453744 kodiert ist.

4. Verfahren nach einem der Ansprüche 1 bis 3, wobei die genetisch veränderte Pflanze eine Sorghum-Pflanze ist oder ausgewählt ist, aus Weizen, Hafer, Mais, Gerste, Roggen und Reis, oder ausgewählt ist, von Abaka, Alfalfa, Mandel, Apfel, Spargel, Banane, Bohne-Phaseolus, Brombeere, Ackerbohne, Raps, Cashew, Cassava, Kichererbse, Zitrus, Kokosnuss, Kaffee, Mais, Baumwolle, Feige, Flachs, Trauben, Erdnuss, Hanf, Kenaf, Lavendel, Mano, Olive, Zwiebel, Erbse, Aschanti, Birne, Perlhirse, Kartoffel, Ramie, Rübsamen, Weidelgras, Sojabohne, Erdbeere, Zuckerrübe, Zuckerrohr, Sonnenblume, Süßkartoffel, Taro, Tee, Tabak, Tomate, Triticale, und Yamswurzel.

5. Verfahren nach einem der Ansprüche 1 bis 4, wobei der grün-bleiben Phänotyp weiter einen Phänotyp einschließt, der ausgewählt ist, aus erhöhter Plastizität der Baumkronenarchitektur, verringerter Baumkronengröße, erhöhter Biomasse pro Blatteinheitsfläche bei der Anthese, höhere Transpirationseffizienz, erhöhter Wasserverbrauch während der Kornfüllung, reduzierte Vor- und Nach-Anthese Biomasseproduktion und verzögerte Seneszenz, oder weiter eine größere Korngröße einschließt.

6. Verwendung eines genetischen Materials, das ein SbPIN4-Protein kodiert, zum Erzeugen, durch rekombinante Mittel, einer genetisch veränderten Pflanze, die Wasser effizienter nutzt als eine nicht genetisch veränderte Pflanze der gleichen Art, wobei die genetisch veränderte Pflanze einen grün-bleiben Phänotyp aufweist, der eine Verschiebung des Wasserverbrauchs zu einer Nach-Anthese-Periode oder eine erhöhte Zugänglichkeit von Wasser während des Pflanzenwachstums oder eine erhöhte Transpirationseffizienz, die in einem erhöhten Ente-Index und Kornertrag unter eingeschränkten Wasserbedingungen resultiert, einschließt, und wobei das SbPIN4-Protein durch Sorghum Gen ID Sb03g037350 von bp 65310051 bis bp 65313194 kodiert ist.

## Revendications

1. Procédé de génération d'une plante génétiquement modifiée qui utilise l'eau plus efficacement qu'une plante non génétiquement modifiée de la même espèce, le procédé comprenant l'introduction par génie génétique à l'aide d'un moyen de recombinaison d'un matériel génétique qui code pour une protéine SbPIN4 afin de conférer un phénotype stay-green, ledit phénotype comprenant un changement d'utilisation de l'eau à la période post-anthèse ou une accessibilité accrue d'eau pendant la croissance des cultures ou une efficacité accrue de la transpiration aboutissant à une augmentation de l'indice de récolte et du rendement en grains dans des conditions où l'eau est limitée, dans lequel ladite protéine SbPIN4 est codée par le gène du sorgho ID Sb03g037350 de pb 65310051 à pb 65313194.

2. Procédé selon la revendication 1, comprenant en outre l'introduction par génie génétique à l'aide d'un moyen de recombinaison d'un matériel génétique qui code pour SbPIN2.

3. Procédé selon la revendication 2, dans lequel ladite protéine SbPIN2 est codée par le gène du sorgho ID Sb03g029320 de pb 57449784 à pb 57453744.

4. Procédé selon l'une quelconque des revendications 1 à 3 dans lequel la plante génétiquement modifiée est un plan de sorgho, ou est sélectionnée parmi le blé, l'avoine, le maïs, l'orge, le seigle et le riz, ou est sélectionnée parmi le chanvre de Manille, la luzerne, l'amande, la pomme, l'asperge, la banane, le haricot phaseolus, la mûre, la fève des marais, le canola, l'anacardier, le manioc, le pois chiche, les agrumes, la noix de coco, le café, le maïs, le coton, la figue, le lin, le raisin, l'arachide, le chanvre, le kenaf, la lavande, le mano, l'olive, l'oignon, le pois, la cacahuète, la poire, le millet à chandelle, la pomme de terre, la ramie, le colza, l'ivraie, le soja, la fraise, la betterave à sucre, la canne à sucre, le tournesol, la patate douce, le taro, le thé, le tabac, la tomate, le triticale et l'igname.

5. Procédé selon l'une quelconque des revendications 1 à 4 dans lequel le phénotype stay-green comprend en outre un phénotype sélectionné parmi une plasticité améliorée de l'architecture de la canopée, une taille réduite de la canopée, une biomasse accrue par unité de surface de feuille à l'anthèse, une efficacité de transpiration plus élevée, une utilisation accrue de l'eau pendant le remplissage des grains, une production réduite de biomasse pré- et post-anthèse et une sénescence retardée, ou comprend en outre une plus grande taille de grain.

6. Utilisation d'un matériel génétique qui code pour une protéine SbPIN4 pour générer grâce à un moyen de recombinaison une plante génétiquement modifiée qui utilise l'eau plus efficacement qu'une plante non génétiquement modifiée de la même espèce, dans laquelle ladite plante génétiquement modifiée présente un phénotype stay-green qui comprend un changement d'utilisation de l'eau à la période post-anthèse ou une accessibilité accrue d'eau pendant la croissance des cultures ou une efficacité accrue de la transpiration aboutissant à une augmentation de l'indice de récolte et du rendement en grains dans des conditions où l'eau est limitée, et dans lequel ladite protéine SbPIN4 est codée par le gène du sorgho ID Sb03g037350 de pb 65310051 à pb 65313194.
